# EUROPEAN PATENT SPECIFICATION

(11) **EP 3 304 511 B1**
(45) Date of publication and mention of the grant of the patent: **28.09.2022**
(21) Application number: 16804188.7
(22) Date of filing: 29.05.2016
(51) Int. Cl.: A61B 5/11, A61B 5/00, G08B 21/22

(54) **PATIENT SUPPORT APPARATUS**
PATIENTENLIEGEVORRICHTUNG
APPAREIL DE SUPPORT DE PATIENT

(30) Priority: 29.05.2015 US 201562168596 P; 01.06.2015 US 201562169270 P; 27.07.2015 US 201562197294 P; 26.08.2015 US 201562210098 P; 17.11.2015 US 201562256233 P; 17.11.2015 US 201562256406 P; 17.11.2015 US 201562256408 P; 26.02.2016 US 201662300340 P
(43) Date of publication of application: 11.04.2018
(73) Proprietor: Hill-Rom Services, Inc., Batesville, IN 47006-9167 (US)
(72) Inventor: ZERHUSEN, Robert Mark, Cincinnati, Ohio 45252 (US); HEIMBROCK, Richard Henry, Cincinnati, Ohio 45238 (US); SHAH, Arpit, Batesville, Indiana 47006 (US); BHAI, Aziz A., Batesville, IN 47006-9167 (US); SMITH, Bradley Thomas, Batesville, IN 47006-9167 (US); WAGNER, Catherine Marie, Osgood, Indiana 47037 (US); LACHENBRUCH, Charles A., Batesville, Indiana 47006 (US); OWSLEY, Clay Gerome, Batesville, IN 47006-9167 (US); TALLENT, Dan R., Hope, Indiana 47246 (US); NACHTIGAL, Daniel, Brookline, Massachusetts 02467 (US); BEDEL, David L., Oldenburg, Indiana 47036 (US); BRZENCHEK, David J., Harrison, Ohio 45030 (US); HITCHCOCK, David J., Westford, Massachusetts 01886 (US); LUBBERS, David P., Cincinnati, Ohio 45248 (US); SEIM, Douglas A., Okeana, Ohio 45053 (US); BORGMAN, Douglas E., Batesville, Indiana 47006 (US); BENZ, Eric David, Sunman, Indiana 47041 (US); IUCHA, Florin, Winter Park, Florida 32792 (US); SAUSER, Frank E., Cincinnati, Ohio 45248 (US); MONSON, Gavin M., Oxford, Ohio 45056 (US); PASCOE, James W., Northborough, Massachusetts 01532 (US); WALKE, James L., Batesville, Indiana 47006 (US); RUDE, Jared, Raleigh, North Carolina 27616 (US); BYERS, John G., Batesville, IN 47006-9167 (US); CHRISTIE, John D., Batesville, Indiana 47006 (US); TURNER, Jonathan D., Dillsboro, Indiana 47018 (US); WILLIAMS, Joshua A., West Harrison, Indiana 47060 (US); LANNING, Karen, Batesville, Indiana 47006 (US); SMITH, Kathryn, Batesville, Indiana 47006 (US); EMMONS, Kirsten M., Batesville, Indiana 47006 (US); BRINKMAN, Mary Kay, Oldenburg, Indiana 47036 (US); BUCCIERI, Michael, Greenfield, Indiana 46140 (US); HIXON, Nathaniel William, Batesville, IN 47006-9167 (US); WIGGERMANN, Neal, Batesville, IN 47006-9167 (US); SCHUMAN, Richard Joseph Sr., Cary, North Carolina 27511 (US); CORBIN, Scott M., Sunman, Indiana 47041 (US); MAMIDI, Sravan, Columbus, Indiana 47201 (US); O'NEAL, Todd P., Fairfield, Ohio 45014 (US); VENTROLA, Todd Steven, Liberty Township, Ohio 45011 (US); PELO, Travis, Batesville, Indiana 47006-9167 (US); OJHA, Unnati, North Carolina 27513 (US); RIBBLE, David Lance, Indianapolis, Indiana 46202 (US); LAWRENCE, Brian L., Batesville, IN 47006-9167 (US); ECKSTEIN, Douglas A., Batesville, Indiana 47006 (US)
(74) Representative: Findlay, Alice Rosemary
(86) International application number: PCT/US2016/034908
(87) International publication number: WO 2016/196403

(56) References cited:
- US-A- 5 664 270
- US-A1- 2011 068 928
- US-A1- 2012 023 670
- US-A1- 2012 089 419
- US-A1- 2012 110 735
- US-A1- 2012 312 196
- US-A1- 2013 219 622
- US-A1- 2014 062 342
- US-A1- 2014 259 410
- US-A1- 2014 259 410
- US-A1- 2014 265 181
- US-A1- 2014 292 529
- US-A1- 2014 323 816
- US-A1- 2014 375 451

## Description

### CROSS-REFERENCE TO RELATED APPLICATIONS

This application claims priority under 35 U.S.C. § 119(e) to U.S. Provisional Application Serial No. 62/168,596, filed May 29, 2015, U.S. Provisional Application Serial No. 62/169,270, filed June 1, 2015, U.S. Provisional Application Serial No. 62/197,294, filed July 27, 2015, U.S. Provisional Application Serial No. 62/210,098, filed August 26, 2015, U.S. Provisional Application Serial No. 62/256,233, filed November 17, 2015, U.S. Provisional Application Serial No. 62/256,406, filed November 17, 2015, U.S. Provisional Application Serial No. 62/256,408, filed November 17, 2015, and U.S. Provisional Application Serial No. 62/300,340, filed February 26, 2016.

### BACKGROUND

The present disclosure relates to patient support apparatuses. More specifically, the present disclosure relates to patient support apparatuses with improved functionality and usability.

There is an ongoing need to reduce the labor required for caregivers to deliver quality patient care. In addition, there is an ongoing need for the cost of healthcare to be reduced. Finally, the comfort of a person in an in-patient environment is directly related to their perception of the quality of their care and their recovery. A patient support apparatus that provides patient comfort, reduced cost, and improved caregiver efficiency addresses these needs.

US2012/023670A1 describes a siderail assembly comprising a siderail body and an indicator. The siderail body includes a handle configured to be gripped by a person. The indicator is configured to emit light to identify the location of the handle.

US2014/265181A1 describes a transport apparatus including a frame, a lift mechanism supporting the frame, and a litter deck for supporting a person. The litter deck is adapted to be reconfigured between a chair configuration and a cot configuration. The litter deck is also adapted to be removably mounted to the frame, with the litter deck being adapted to be supported by the frame while the litter deck is in its chair configuration and also while being reconfigured between its chair configuration and its cot configuration.

US2012/110735A1 describes a siderail assembly including a guide, a support frame coupled to the frame and movable between first and second positions, and a barrier coupled to the support frame and movable therewith. The siderail assembly further includes a handle coupled to the barrier to move between a first position and a second position relative to the barrier. The siderail assembly may include electronic controls to change the position or limit movement of various portions of a patient support apparatus on which the siderail assembly may be coupled.

US2014/0259410A1 describes a patient support apparatus including an alert light assembly or an alert light module having separate zones that are individually illuminated to convey information regarding respective alert conditions. The zones each have indicia related to a particular condition of the patient support apparatus. The illuminated zones are each sufficiently large so as to be seen from afar, such as on the order of ten feet or more.

### SUMMARY

The invention is set out in the appended set of claims.

According to a first aspect of the present disclosure, a patient support apparatus comprises a controller, a plurality of sensors coupled to the controller, and a notification system. The plurality of sensors coupled to the controller are each operable to provide a signal to the controller indicative of the status of a patient position monitoring system of the patient support apparatus. The patient support apparatus also comprises a frame, a barrier, a control system and a user interface. The barrier comprises a handle and is supported by the frame and is movable vertically relative to the frame. The handle has a plurality of light emitting diodes positioned within the handle such that emissions from the light emitting diodes are visible external to the handle. The notification system is coupled to the controller. The notification system is operable to process signals from the controller which provide an indication of the status of the patient position monitoring system compared to established acceptable operating conditions, and, if the status of the patient position monitoring system deviates from the established acceptable operating condition for the patient position monitoring system, provide a visual indication of the deviation by illuminating the handle in a first manner if the status of the patient position monitoring system is within the acceptable operating condition and illuminating the handle in a second manner if the status of the patient position monitoring system is outside of the acceptable operating condition. A visual indication of the status of the position of a patient is provided by illuminating the handle.

In some embodiments, the notification system is operable to project the first iconic representation to a surface spaced apart from the patient support apparatus.

In some embodiments, the first iconic representation is simultaneously illuminated on a surface of the patient support apparatus and projected onto the surface spaced apart from the patient support apparatus.

In some embodiments, the first iconic representation is projected to the surface spaced apart from the patient support apparatus by a projector located on the patient support apparatus.

In some embodiments, illuminating the first iconic representation in a first manner comprises illuminating the first iconic representation in a first color and illuminating the first iconic representation in a second manner comprises illuminating the first iconic representation in a second color.

In some embodiments, providing the visual indication of the deviation includes simultaneously illuminating a first iconic representation of the component on a surface of the patient support apparatus in a first color and projecting the first iconic representation of the component on the surface spaced apart from the patient support apparatus in the first color.

In some embodiments, providing the visual indication of the lack of a deviation includes simultaneously illuminating a first iconic representation of the component on a surface of the patient support apparatus in a second color and projecting the first iconic representation of the component on the surface spaced apart from the patient support apparatus in the second color.

In some embodiments, providing the visual indication of the deviation includes simultaneously illuminating a first iconic representation of the component on a surface of the patient support apparatus in a first color and projecting the first iconic representation of the component on the surface spaced apart from the patient support apparatus in the first color.

In some embodiments, providing the visual indication of the lack of a deviation includes simultaneously illuminating a first iconic representation of the component on a surface of the patient support apparatus in a second color and projecting the first iconic representation of the component on the surface spaced apart from the patient support apparatus in the second color.

In some embodiments, the surface spaced apart from the patient support apparatus is the surface of a floor, the first iconic representation being projected to a position that is not directly below any portion of the patient support apparatus.

### BRIEF DESCRIPTION OF THE DRAWINGS

The detailed description particularly refers to the accompanying figures in which:
Fig. 1 is a perspective view from a patient's left side of a patient support apparatus illustratively embodied as a hospital bed;
Fig. 2 is another perspective view of the patient support apparatus of Fig. 1;
Fig. 3 is a perspective view of the patient support apparatus of Fig. 1, the patient support apparatus including a patient support surface illustratively embodied as a mattress positioned on the hospital bed;
Fig. 4 is a plan view of the patient support apparatus of Fig. 3 as viewed from the patient's left side of the patient support apparatus with the siderails of the hospital bed in a lowered position;
Fig. 5 is a perspective view of the patient support apparatus of Fig. 3 with a head section and a thigh section of a deck of the patient support apparatus being raised;
Fig. 6 is an exploded assembly view of a top portion of a foot deck section of a patient support apparatus;
Fig. 7 is an exploded assembly view of a bottom portion of a foot deck section of a patient support apparatus, the foot deck section having an actuator two power extension retraction of the foot deck section;
Fig. 8 is exploded assembly view of a portion of a notification system supported on the end of the foot deck section of a patient support apparatus, the notification system operable to provide a visual indication of the status of components of the patient support apparatus;
Fig. 9 is a perspective view of a projection structure of the notification system of Fig. 8, the projection structure including a slide that includes an iconic image that is projected by the projection structure to a surface spaced apart from the patient support apparatus;
Fig. 10 is an exploded assembly view of an embodiment of a right head side rail suitable for use with the patient support apparatus of Fig. 1;
Fig. 11 is exploded assembly view of an embodiment of a left head side rail suitable for use with the patient support apparatus of Fig. 1;
Fig. 12 is a perspective view of a patient support apparatus including a hospital bed and an adjacent chair, the chair having an exit sensor that communicates with the hospital bed;
Fig. 13 is a plan view of a side rail of the patient support apparatus of Fig. 1;
Fig. 14 is a diagrammatic representation of the menu structure is presented on a graphical user interface;
Fig. 15 is a screenshot of a home screen of the menu structure of Fig. 14;
Fig. 16 is a screenshot of a of a home screen displayed on a graphical user interface when the patient support apparatus is on battery power;
Fig. 17 is a screenshot of a another embodiment of a home screen;
Fig. 18 is an illustration of the various functions available within the menu structure of Fig. 14;
Fig. 19 is an exploded assembly view of a side rail including a grip may be illuminated in response to conditions on the patient support apparatus;
Fig. 20 is a view of a side rail including a grip that may be illuminated, the grip and not illuminated;
Fig. 21 is a view similar to Fig. 20, with the grip illuminated;
Fig. 22 is a partial view of a grip of a side rail that in includes an illuminated indicator;
Fig. 23 is a view similar to Fig. 22, Fig. 23 illustrating the grip being illuminated;
Fig. 24 is a perspective view of a portion of a patient support apparatus including an indicator system for illuminating images on a surface spaced apart from the patient support apparatus;
Fig. 25 is a plan view of an indicator system positioned on the foot end of a foot deck section of a patient support apparatus;
Fig. 26 is a diagrammatic representation of the illumination system used in the indication system of Fig. 24;
Fig. 27 is a diagrammatic representation taken from the side of a foot deck section of a patient support apparatus showing the projection of indicators by the system of Fig. 24;
Fig. 28 is a partial exploded assembly view of a side rail for the patient support apparatus of Fig. 1, the side rail having a cavity for receiving a light strip that is operable to illuminate the grip of the side rail;
Figs. 29-33 are detailed views of the light strip of Fig. 28;
Figs. 34-70 are a series of screenshots of screens for a graphical user interface of the patient support apparatus of Fig. 1, the screenshots associated with the alerts portion of the menu structure of Fig. 14;
Figs. 71-88 are a series of screenshots for a graphical user interface of the patient support apparatus of Fig. 1, the screenshots associated with the Bluetooth^{®} function of the menu structure of Fig. 14; and

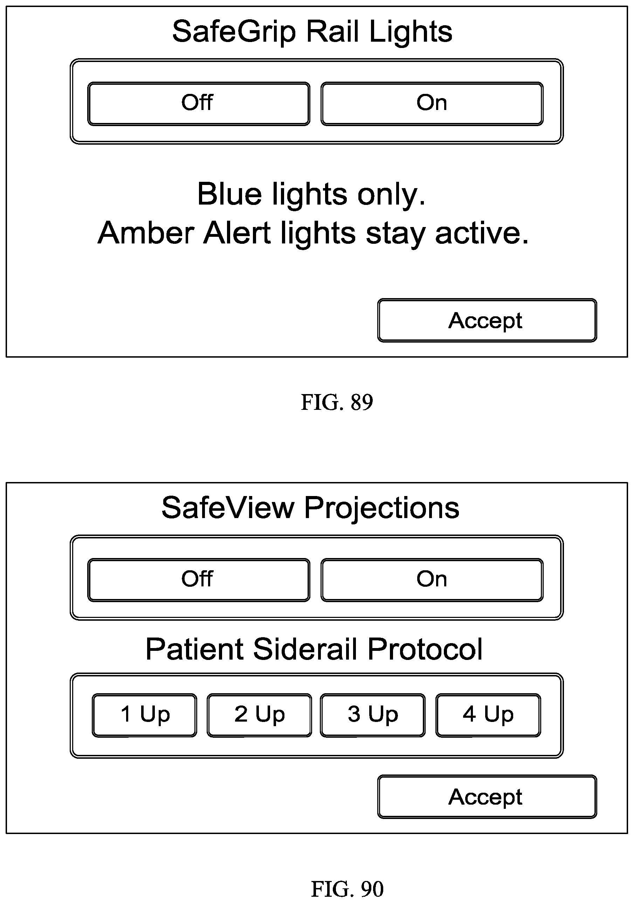

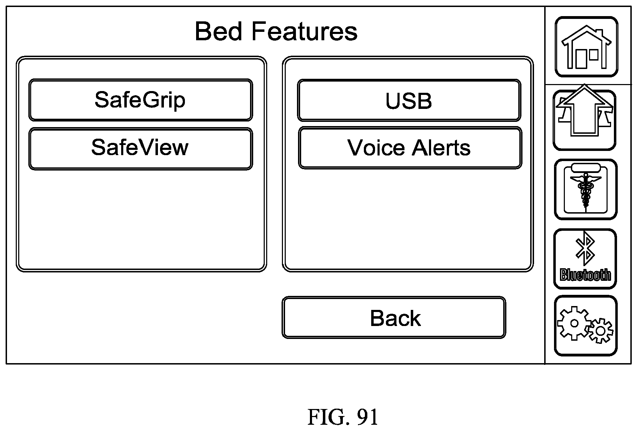

Figs. 89-91 are a series of screenshots for graphical user interface of the patient support apparatus of Fig. 1, the screenshots associated with the preferences function of the menu structure of Fig. 14.

### DETAILED DESCRIPTION OF THE DRAWINGS

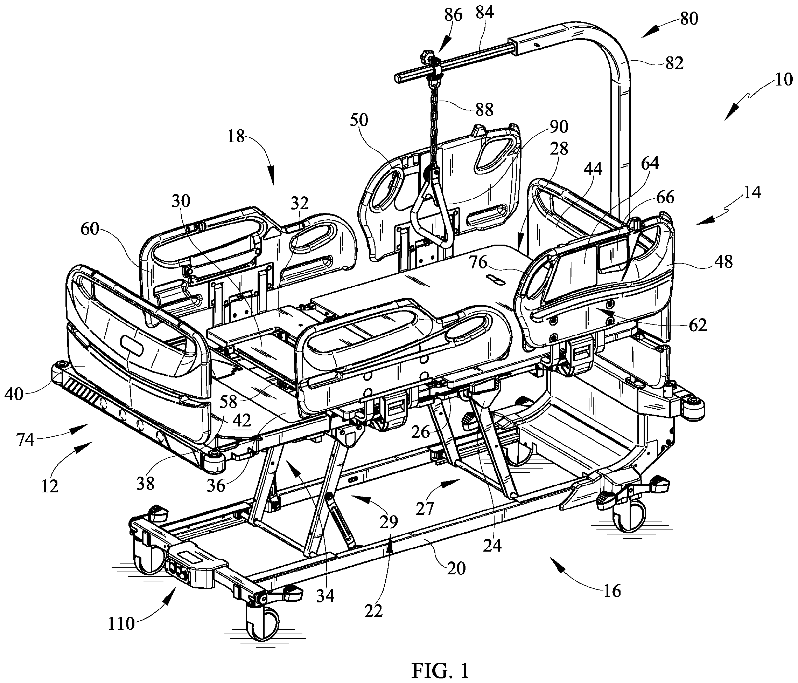

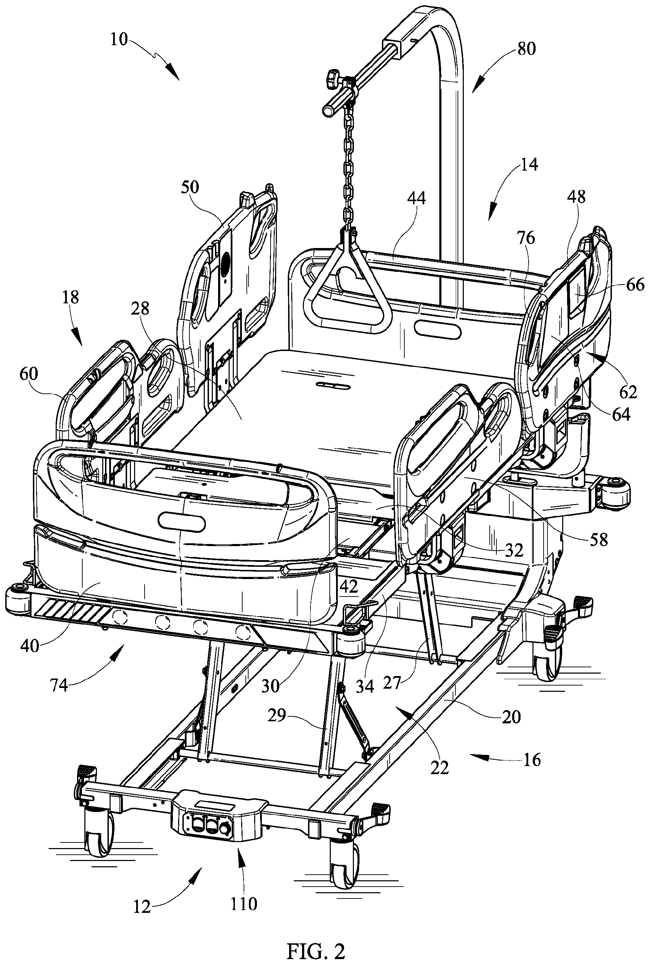

Referring to Figs. 1-4, a patient support apparatus 10 is illustratively embodied as a hospital bed 10. The views shown in Figs. 1-3 are generally taken from a position that is oriented at the left side, foot end of the hospital bed 10. For purposes of orientation, the discussion of the hospital bed 10 will be based on the orientation of a patient supported on the hospital bed 10 in a supine position. Thus, the foot end 12 of the hospital bed 10 refers to the end nearest the patient's feet when the patient is supported on the hospital bed 10 in the supine position. The hospital bed 10 has a head end 14 opposite the foot end 12. A left side 16 refers to the patient's left when the patient is lying in the hospital bed 10 in a supine position. The right side 18 refers to the patient's right. When reference is made to the longitudinal length of the hospital bed 10, it refers a direction that is represented by the lines that generally extend between the head end 14 and foot end 12 of the hospital bed 10. Similarly, lateral width of the hospital bed 10 refers to a direction that is represented by the lines that generally extend between the left side 16 and right side 18.

The hospital bed 10 includes a base frame 20 which supports a lift system 22. The lift system 22 engages the base frame 20 and an upper frame 24 such that the lift system 22 moves the upper frame 24 vertically relative to the base frame 20. The lift system 22 includes a head end linkage 27 and a foot end linkage 29. Each of the linkages 27 and 29 are independently operable and may be operated to cause the hospital bed 10 to move into a tilt position which is when the head end 14 of the upper frame 24 is positioned lower than the foot end 12 of the upper frame 24. The hospital bed 10 may also be moved to a reverse tilt position with the foot end 12 of the upper frame 24 is positioned lower than the head end 14 of the upper frame 24.

The upper frame 24 supports a load frame 26. The load frame 26 supports a head deck 28 which is movable relative to the load frame 26. The load frame 26 also supports an articulated seat deck 30, also movable relative to the load frame 26 and a fixed seat deck 32. Also supported from the load frame 26 is a foot deck 34 that is articulated and moveable relative to the load frame 26. The foot deck 34 in the illustrative embodiment of Figs. 1-4, provides for powered pivoting of the foot deck 34 and manual extension and retraction of the foot deck 34 to vary the length of the foot deck 34. In other embodiments, powered pivoting of the foot deck 34 may be omitted and the related movement may be caused manually, or follow movement of the articulated seat deck 30. In addition, in some embodiments, extension and retraction of the foot deck 34 may be powered by an actuator.

The foot deck 34 includes a first portion 36 and a second portion 38, which moves relative to the first portion 36 to vary the size of the foot deck 34. The second portion 38 moves generally longitudinally relative to the first portion 36 to vary the longitudinal length of the foot deck 34 and, thereby, the longitudinal length of the hospital bed 10.

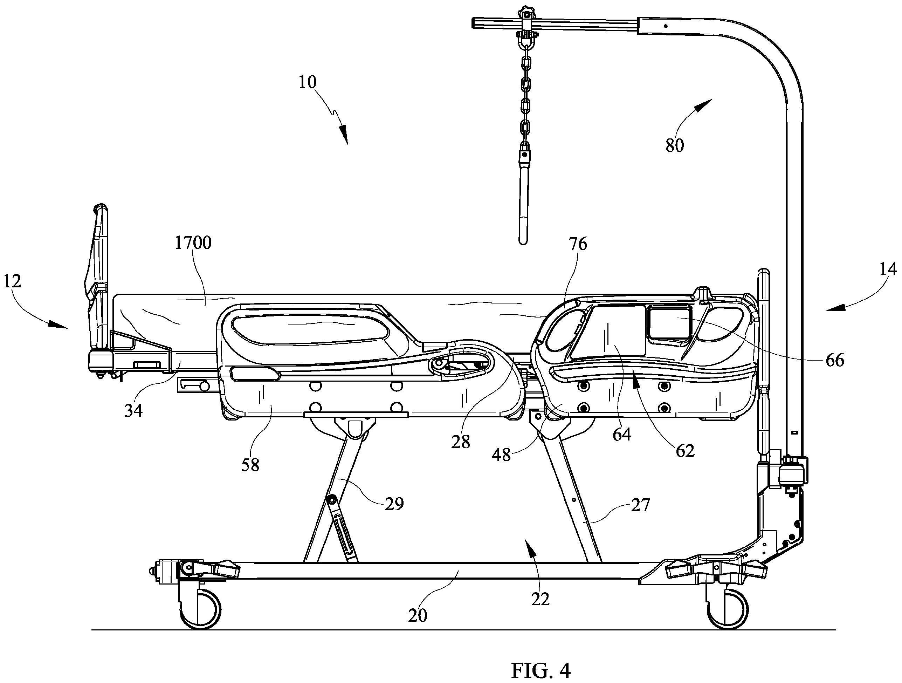

A foot panel 40 is supported from the second portion 38 and extends vertically from an upper surface 42 of the second portion 38 to form a barrier at the foot end 12 of the hospital bed 10. A head panel 44 is positioned on an upright structure of the base frame 20 and extends vertically to form a barrier at the head end 14 of the hospital bed 10. A left head siderail 48 is supported from the head deck 28 and is moveable between a raised position shown in Fig. 1 and a lowered position shown in Fig. 4. A right head siderail 50 is also moveable between the raised position of Fig. 1 and lowered position similar to that of the left head siderail 48 in Fig. 4. As shown in Fig. 1, in the raised position, the siderails 48 and 50 extend above an upper surface 42 of the respective decks of the hospital bed 10 when the siderails 48 and 50 are in a raised position.

The hospital bed 10 also includes a left foot siderail 58 and a right foot siderail 60, each of which is supported directly from the load frame 26. Each of the siderails 48, 50, 58, and 60 are operable to be lowered to a position below the upper surface 42. It should be noted that when the head deck 28 is moved, the head siderails 48 and 50 move with the head deck 28 so that they maintain their relative position to the patient. This is because both of the head siderails 48 and 50 are supported by the head deck 28.

Referring to the left head siderail 48, a user interface 62 includes a hard panel 64 and a graphical user interface 66. The user interface 62 will be discussed in further detail below, but it should be understood that the hard panel 64 provides indications to a user regarding the status of certain functions of the hospital bed 10 as well as providing a standard set of fixed input devices. The graphical user interface 66 includes a touchscreen display that provides information to a user as well as allowing for flexible, menu driven, operation of certain functions of the hospital bed 10. In other embodiments, the right head siderail 50 may include a second graphical user interface duplicative of the graphical user interface 66.

Additional information is provided to a caregiver through an optional indicator panel 74 which displays the status of various conditions of the hospital bed 10 graphically to a caregiver at the foot end 12 of the hospital bed 10. The location of the indicator panel 74 makes the statuses of the conditions easily discernable from a distance, such that a caregiver may quickly ascertain the statuses from the hallway or the door of a patient's room. As will be discussed below, additional indication of the statuses may be projected on the floor under the foot end 12 of the hospital bed 10, providing larger images on the floor, making the images more easily discerned by a caregiver. Similarly, an illuminated grip 76 is positioned on the left head siderail 48, the illuminated grip 76 being selectively illuminated in different colors to provide an indication of the status of one or more functions of the hospital bed 10 to a caregiver. Similarly, the right head siderail 50 also includes an illuminated grip, which is duplicative of the illuminated grip 76.

As shown in Figs. 1-4, the hospital bed 10 includes a patient helper 80, which is supported from the base frame 20. The patient helper 80 includes a curved arm 82 that is fixed to the base frame 20 with a support arm 84 extending from the curved arm 82. The support arm 84 is formed to include a hexagonal cross-section which provides a resistance to rotation of a clamp 86 when the clamp 86 is secured to the support arm 84. The clamp 86 supports a chain 88 which depends vertically from the clamp 86. The chain 88 supports a grip 90 which is graspable by a patient positioned in a supine position on the hospital bed 10 so that the patient may use the patient helper 80 to reposition themselves in the hospital bed 10.

The hospital bed 10 also includes an auxiliary outlet 110 positioned at a foot end 12 of the base frame 20. The auxiliary outlet 110 provides a separate circuit, independent of the electrical system of the hospital bed 10, which may be used to power accessory equipment positioned at the foot end 12 of the hospital bed 10.

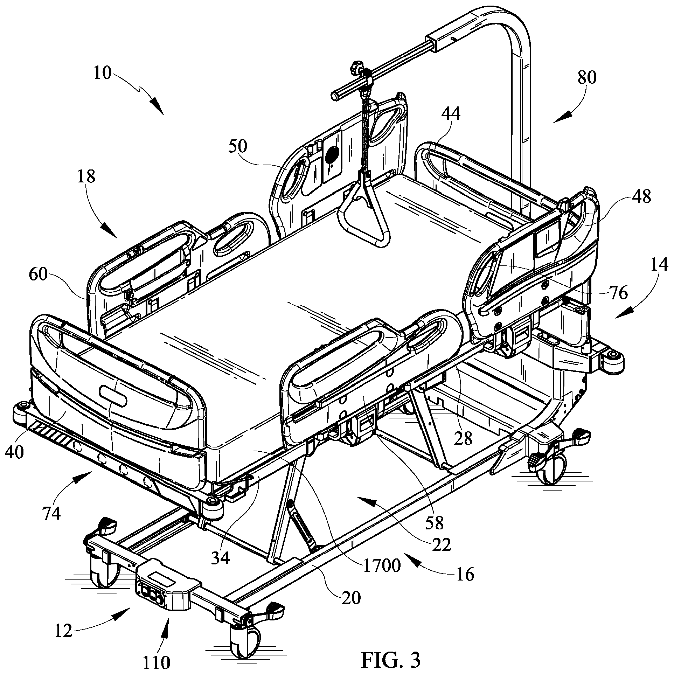

The hospital bed 10 is configured to support a patient support surface 1700 (see Fig. 3). The control system of the hospital bed 10 is configured to interact with several subsystems and auxiliary devices, permitting the user of the hospital bed 10 to control or interact with the subsystems through the graphical user interface 66. For example, the graphical user interface 66 allows a user to control operation of an optional pneumatic patient support surface. A user may also interact with the indicator panel 74 and the illuminated grips 76 to define the conditions that cause each of those devices to provide indications to a user. The hospital bed 10 also includes a scale system with the graphical user interface 66 providing the interface for the user to the operation of the scale system and associated operations and alerts. Still further, the hospital bed 10 may include a patient position monitoring function that is operated from the graphical user interface 66. Other subsystems and accessories that may be interfaced with the graphical user interface 66 include a chair exit monitoring system, a sequential compression device, a radio frequency based authentication system for identifying appropriate caregivers, a charting function that allows a user to chart certain information to the patient's electronic medical record from the graphical user interface 66. In addition, the hospital bed 10 may optionally be configured with an incontinence detection system which provides an alert if the patient has an incontinent event. Each of these functions and accessories may employ the graphical user interface 66 to configure and monitor the various subsystems and accessories. Utilization of the graphical user interface 66 permits optional functions and accessories to be added without the need for reconfiguring any hard keys on the hospital bed 10.

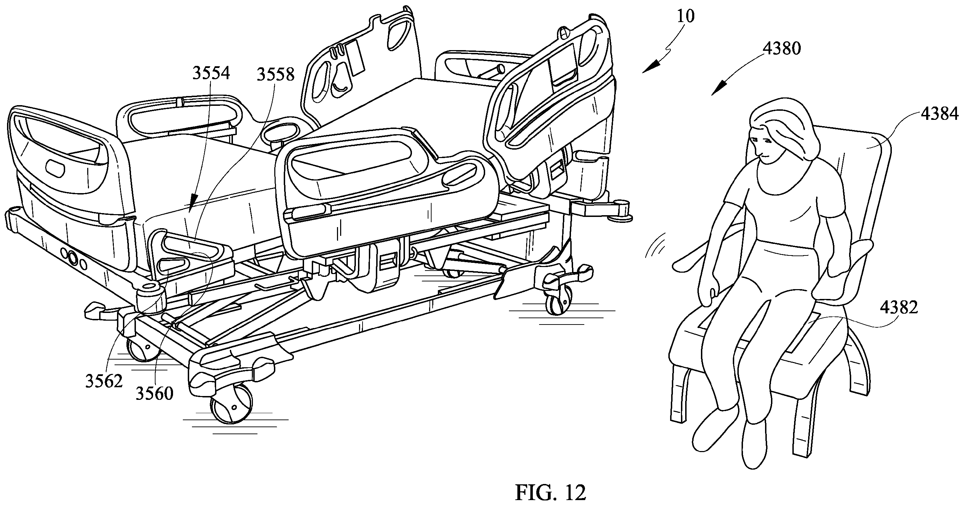

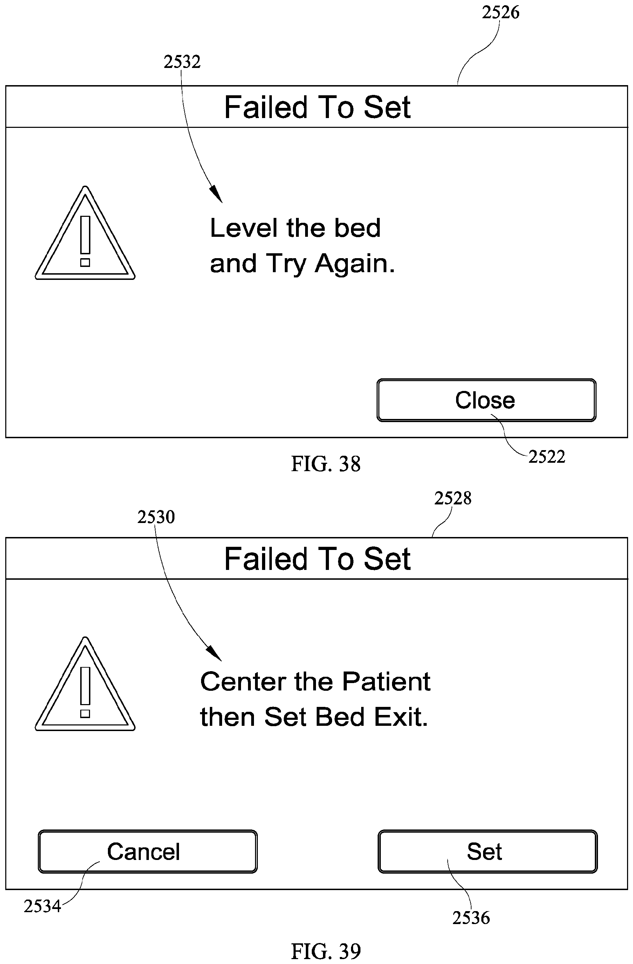

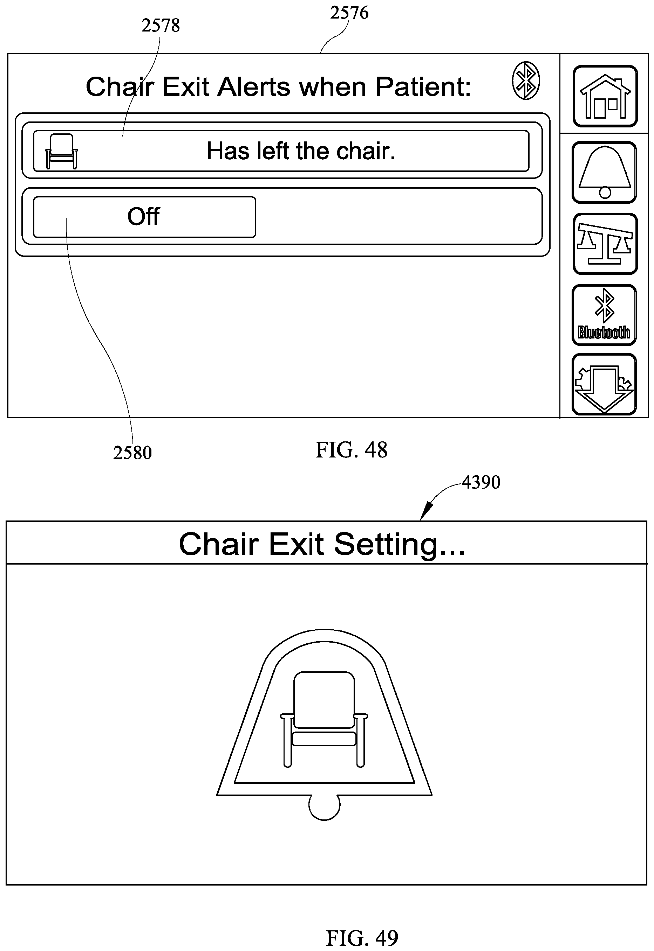

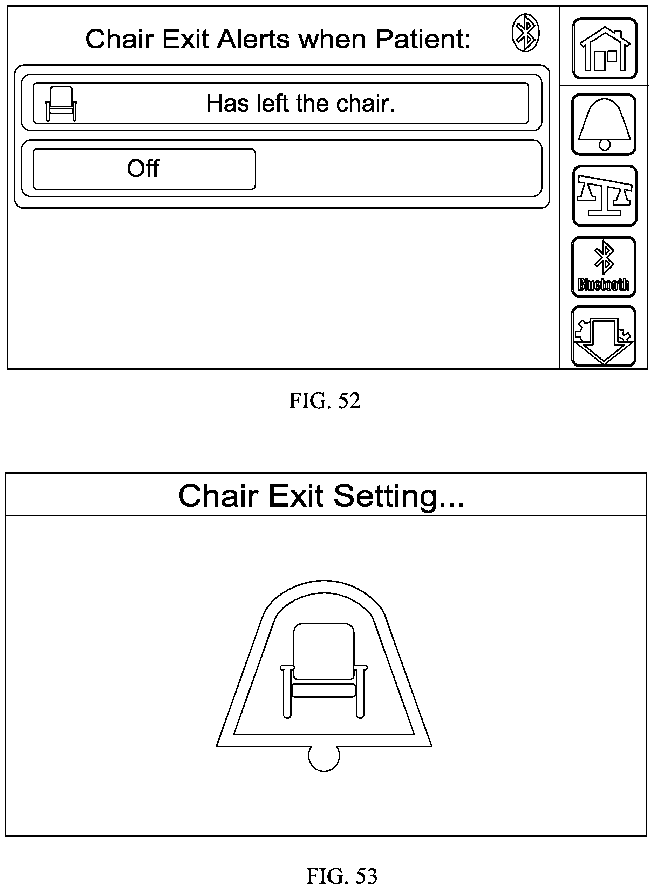

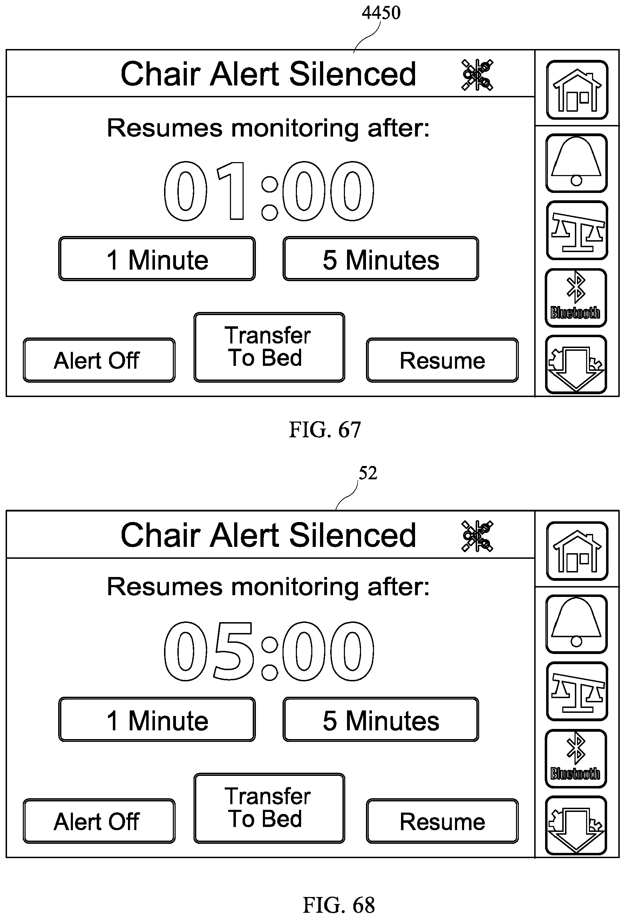

For example, referring now to Fig. 12, the patient support apparatus 10 may be configured to be part of a system 4380 which includes the patient support apparatus 10 and a detector 4382 configured to be positioned on a chair 4384 to be used by a patient. The detector 4382 is operable to communicate wirelessly with the patient support apparatus 10 such that the detector 4382 is integrated with the patient position monitoring system of the patient support apparatus 10. In some embodiments, when the patient sits on the detector 4382, the system automatically arms to monitor for an egress from the chair 4384 by the patient. If an egress condition is detected, the detector 4382 indicates that condition to the patient support apparatus 10 which then alerts a caregiver via the patient position monitoring system of the patient support apparatus 10. For example, as shown in Fig. 48, a caregiver may use the graphical user interface to set the patient position monitoring system between one of three detection settings: detecting when a patient changes position; detecting when the patient moves toward the edge of the patient support apparatus 10; or detecting when the patient has left the patient support apparatus. The patient position monitoring system may be programmed with a voice prompt or other auditory alarm or alert encouraging the patient to stay in the patient support apparatus 10 until assistance arrives. In some embodiments, the voice prompt will in courage the patient to "please stay in hospital bed 10." Further details of the operation of the patient position monitoring system and chair exit alarms is shown in Figs. 39-70.

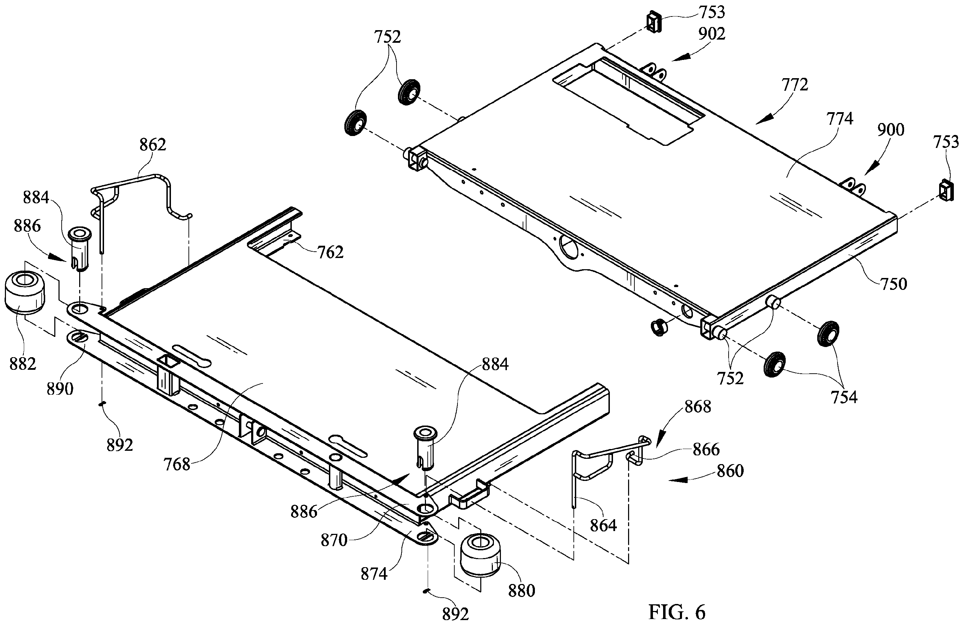

As shown in Figs. 6-7, the foot deck 34 shown to include the first portion 36 and the second portion 38 which moves relative to the first portion to extend and retract the length of the foot deck 34. Extension and retraction of the foot deck 34 is controlled by an actuator 730 which is fixed to the first portion 36. The actuator 730 includes a body 732, a rod 734, and a rod end 736. The rod end 736 is pinned to the second portion 38. The actuator 730 includes an end 738 which is pinned to a yoke 740 on the first portion 36 and secured by a pin 742 and retaining clip 744. When the actuator 730 is in a retracted position, such as that shown in Fig. 6, the foot deck 34 is fully retracted with its length minimized. Extension of the actuator 730 drives the second portion toward the foot end 12 of the foot deck 34 to extend the length of the foot deck 34 and, thereby, the length of the hospital bed 10.

The first portion 36 includes a frame 746 with laterally space rails 748 and 750. Each of the rails 748 and 750 have two axles 752 positioned on the outboard sides of the rails 748 and 750 which are capped with a pair of caps 753, 753. The second portion 38 includes a pair of guides 751 positioned in the end of channels 758 and 760 that engage the rails 748, 750 of first portion 36 to guide second portion 38 as it moves relative to first portion 36. The first portion includes a pair of rollers 754 on each side, each of the rollers 754 supported on an axle 752. The second portion 38 includes a frame 756 which has a pair of laterally spaced channel members 758 and 760. When the second portion 38 is engaged with the first portion 36, the rollers 754 are retained on the axles 752 by the engagement of the rollers 754 with the respective channels 762 and 764 of the channel members 758 and 760.

The second portion 38 is supported on the first portion 36 by the interaction of the rollers 754 with the channels 762 and the interaction of the rails 748, 750 of first portion 36 with the guides 751, 751. The second portion 38 includes a deck panel 766 which spans the distance between the channel members 760 and 762 define an upper support surface 768. The first portion 36 also includes a deck panel 772 which has an upper support surface 774. When the second portion 38 is engaged with the first portion 36, a portion of the deck panel 766 overlies a portion of the deck panel 772. Further support for the engagement between the first portion 36 and the second portion 38 is provided by three glide members 776 which are secured to a lower surface 770 of the deck panel 766. The glide members 776 are secured to the surface 770 by an adhesive and are positioned to engage the upper surface 774 of the deck panel 772 of the first portion 36. The glide members act as bearings between the deck panel 766 and deck panel 772 during extension and retraction of the foot deck 34.

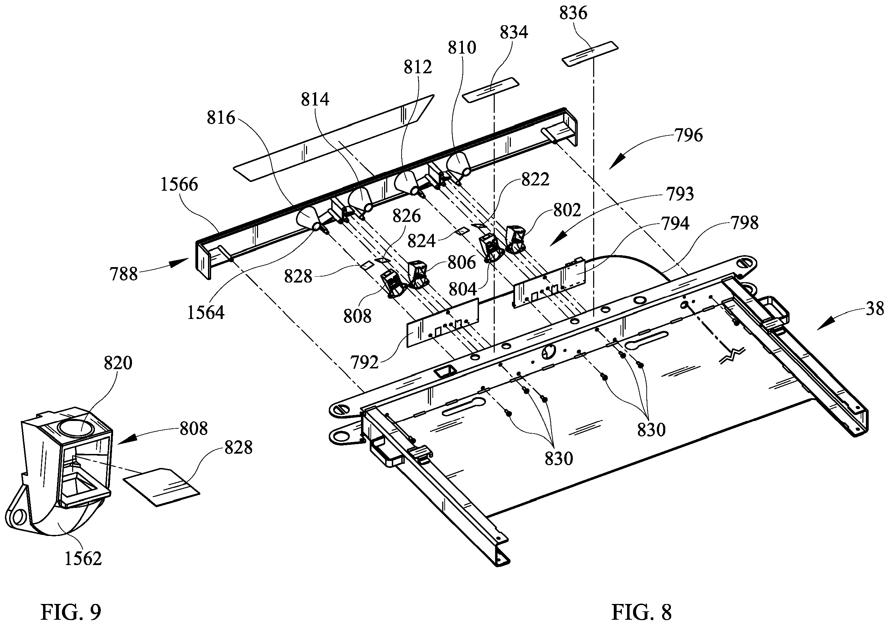

The rod end 736 of the actuator 730 is connected to a yoke 780 formed on the second portion 38 by a pin 782 and a retaining clip 784. The yoke 780 is formed in a channel member 786 positioned at the foot end 12 of the second portion 38. The channel member 786 is open toward the foot end 12 to define a space in which electrical indicator components may be positioned. The electrical components are enclosed by a cover 788 which is secured to the base frame 20 by six fasteners 790. The electrical components are best seen Fig. 8 and include a pair of circuit boards 792 and 794 which are configured to generate indicators of the status of certain conditions of the hospital bed 10 as will be discussed in further detail below.

The circuit boards 792 and 794 are a part of an indicator system 796 that provides detailed information to a caregiver regarding the status of the hospital bed 10 and a patient supported on the hospital bed 10. The circuit boards 792 and 794 receive information over a cable 798 that is connected to the control system of the hospital bed 10. Circuit board 792 is connected to the circuit board 794 by another cable 800. The circuit boards 792 and 794 include logic which processes the information provided over the cable 798 to cause the indicator system 796 to provide an indication of the status of components of the hospital bed 10. In the illustrative embodiment, the indicator system provides information regarding the status of a hospital bed 10 exit system of the hospital bed 10, an indication as to whether or not the hospital bed 10 is in its lowest position, and an indication as to whether not all of the side rails 48, 50, 58, and 60 are in their raised position.

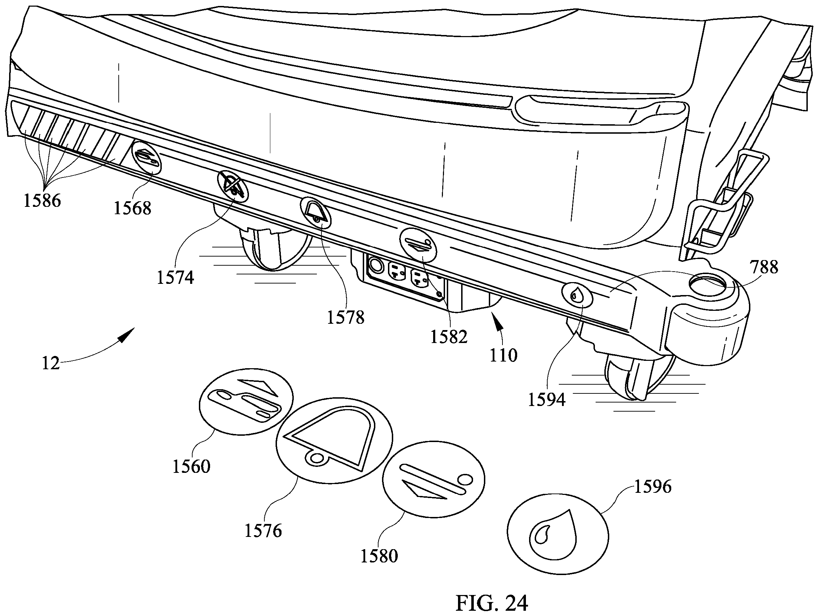

Indication of the statuses may be projected onto the floor below the foot deck 34 by one of four projectors 802, 804, 806, or 808. For example, the projector 808 is associated with the indication as to whether or not all of the side rails 48, 50, 58, and 60 are in their raised position. When active, the projector 808 projects an image such as the image 1560 shown on the floor in Fig. 24. The image 1560 may be projected in either a green or amber color. To project the image 1560, the projector 808 is mounted over a pair of LEDs mounted on the circuit board 792, with one of the LEDs illuminating in an amber color and the other of the LEDs illuminating a green color. When one or the other of the two LEDs is illuminated, the light is conducted through the projector 808 and through the slide 828 positioned in the projector 808. The projector 808 is shown in further detail in Fig. 9 where the slide 828 is positioned in the projector 808, light is transmitted through a body 1562 of the projector 808 and a lens 820 which controls the focus of the image 1560 on the floor. If the control system of the hospital bed 10 provides a signal to the logic of the indication system 796 that one or more of the siderails 48, 50, 58, and 60 are not in the raised position, the amber LED associated with projector 808 would be illuminated so that the image 1560 would be illuminated in an amber color.

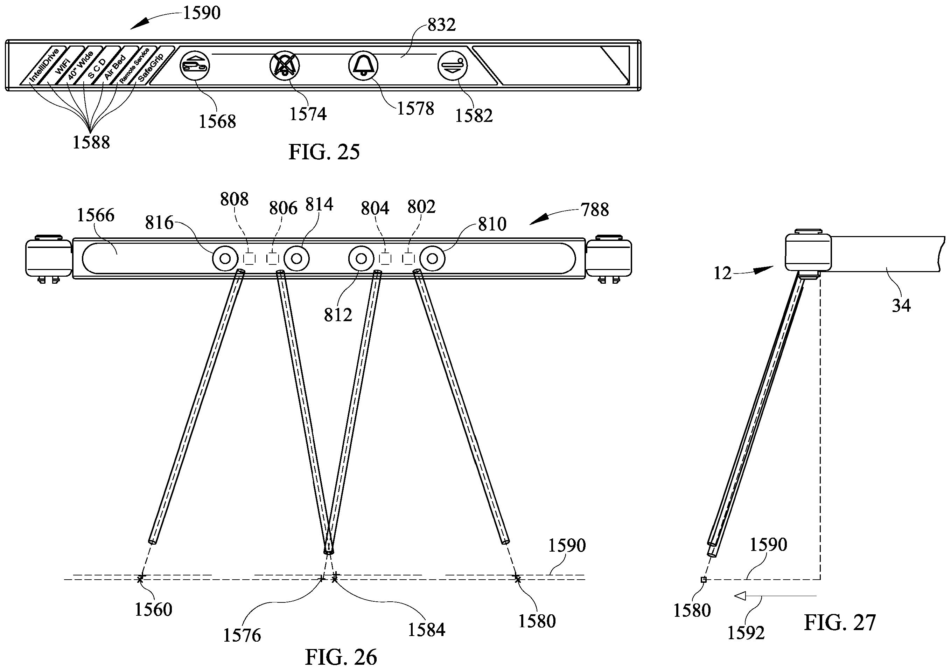

The indication system 796 also includes a lamp 816 which has a frusto-conical shape with an end 1564 that is configured to overlie another pair of LEDs on the circuit board 792. The lamp 816 is configured to direct light from the associate LEDs to an outer surface 1566 of the cover 788. Referring again to Fig. 24, the lamp 816 is associated with an indicator 1568. The indicator 1568 is part of an overlay 1570 positioned on the cover 788. The overlay 1570 is configured to position certain indicia over the openings of the various lamps, such as the opening 1572 for lamp 816 as shown in Fig. 26. Thus, when the lamp 816 is illuminated by the LEDs, the light from the LEDs is transmitted through the indicator 1568. The logic that determines whether or not one or more of the siderails 48, 50, 58, 60 are in their raised position also controls the operation of the LEDs associated with the indicator 1568 and lamp 816. The control system of the hospital bed 10 is operable to operate the indication system 796 to illuminate the indicator 1568 and the image 1560 each provide the status of the siderails 48, 50, 58, 60 simultaneously. The control system may also be configured to illuminate the indicator 1568 only without projecting the image 1560, or project only the image 1560 without illuminating the indicator 1568.

The projector 806 utilizes a slide 826 to illuminate an image 1584 on the floor that is similar to the icon shown as an indicator 1574 shown in Fig. 24. The indicator 1574 and an accompanying image 1584, which is not shown in detail, provide an indication that a patient position monitoring system of the hospital bed 10 is not armed. The indicator 1574 is illuminated by a lamp 814. Projector 806 and lamp 814 engage the circuit board 792 in a manner similar to that of projector 808 and lamp 816. When the image 1584 or indicator 1574 are illuminated green, it provides an indication that the patient position monitoring system is not armed and that patient position monitoring is not indicated for the patient associated with the hospital bed 10. This may rely on information entered into the hospital bed 10 controller by a caregiver, or may be gathered by the control system from an electronic medical record system of the hospital. If the indicator 1574, or the associated image 1584, is illuminated amber, a caregiver will know that the patient position monitoring system is not armed but that the patient has indications that a support protocol that requires the use of the patient position monitoring system.

A projector 804 engages LEDs on the circuit board 794 and projects an image 1576 by way of a slide 824. The image 1576 is projected when the patient position monitoring system is armed. Similarly an indicator 1578 is illuminated by a lamp 812. When the image 1576 and/or the indicator 1578 are green, it provides an indication that the patient position monitoring system is armed and that no alarm condition has been detected. On the other hand, if the image 1576 and/or the indicator 1578 are presented in an amber color, it provides an indication that the patient position monitoring system is armed and an alarm condition exists.

A projector 802 projects light through a slide 822 to present an image 1580 that conveys the status of the hospital bed 10 position. Referring to Fig. 24, when the hospital bed 10 is in its lowest position, the image 1580 is projected in green while an amber color indicates that the hospital bed 10 is not in its lowest position. Similarly, a lamp 810 (seen in Fig. 8) conducts light to an indicator 1582 the same logic as applied to the image 1580 regarding the appropriate color.

A standard overlay 832 is positionable on the surface 1566 as shown in Fig. 24. The cover 788 includes a number of channels 1586 positioned on the right side 18 of the foot deck 34. The channels are sized to receive one or more labels 1590 that include various indicia 1588 that provide information to a user as to the configuration of the hospital bed 10. The labels 1590 provide a quick reference for caregiver to identify the options present on the particular hospital bed 10.

Referring now to Fig. 26, it is shown how the arrangement of the lamps 802, 804, 806, 808 are capable of projecting the various images 1580, 1584, 1576, 1560 onto a surface 1590 of the floor. Because the images 1576 and 1584 are mutually exclusive, the lamps 806 and 804 are arranged projector images at the same point. Fig. 27 shows how the images 1580, 1584, 1576, 1560 are projected at a position that is not directly vertically below the foot end 12 of the head deck 34, but are spaced horizontally a distance 1592. The deviation of the images 1580, 1584, 1576, 1560 outwardly from a position directly below the foot deck 34 assures that the images will be visible when the hospital bed 10 is in its lowest position and a caregiver's view of the images 1580, 1584, 1576, 1560 is not obstructed.

As shown in Fig. 24, the overlay 1570 is similar to the overlay 832 of Fig. 25, however the overlay 1570 includes an additional indicator 1594 and the notification system of the embodiment of Fig. 24 is capable of projecting an image 1596. In the embodiment of Fig. 24, the indicator 1594 and image 1596 provide notification to a caregiver of the status of an incontinence detection system. Following the approach used above, when the indicator 1594 or image 1596 is presented in a green color, it is indicative that an incontinence system is active and no alarm conditions exist. However if the indicator 1594 and/or image 1596 are presented in an amber color, it provides an indication to a caregiver that the incontinence detection system is active and an alarm condition exists.

It is contemplated that each of the monitored conditions would be independently configurable by a caregiver. For example, one or more of the indicators 1568, 1574, 1578, 1582, or 1594 may be deactivated so that the particular condition is not indicated and the indicator remains dormant and not illuminated. As explained above, the projector's 802, 804, 806, 808 may be deactivated such that the caregiver only relies upon the indicators 1568, 1574, 1578, 1582, or 1594 for an indication of the status by the notification system 796.

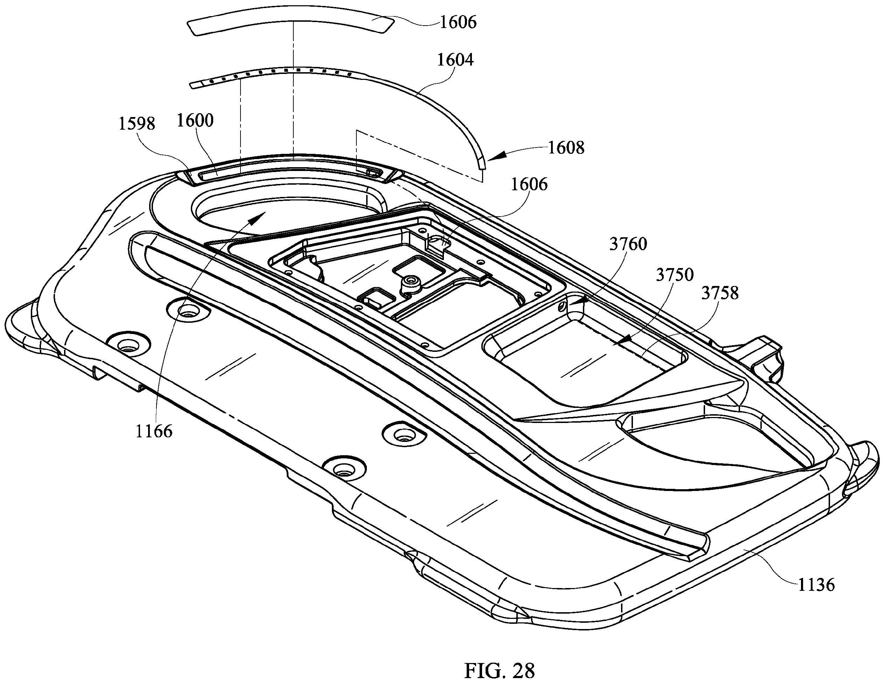

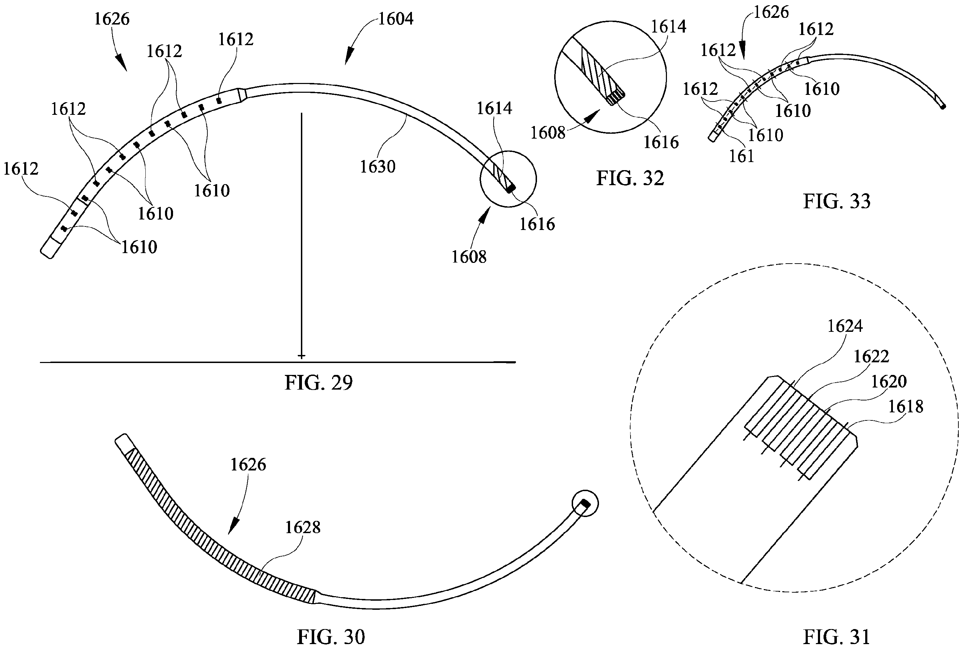

The head end side rails 48 and 50 are configurable to provide additional indications of the status of components of the hospital bed 10 under the control of the notification system 796 by illuminating the grip 1166 of the head siderails 48, 50. In the embodiment of Fig. 29, the body 1136 of side rail 48 has a depression 1598 formed on the outboard side of the grip 1166 and a channel 1600 formed in the interior of the grip 1166. In the embodiment of Fig. 29, an insert 1602 is positioned in the depression 1598 to fill in the missing contour of the grip 1166. In another embodiment shown in Fig. 28, a light strip 1604 is positioned in the channel 1600 and a translucent overlay 1606 is positioned over the light strip 1604. The cavity 1600 is in communication with an outlet 1606 through which an end 1608 is fed to connect to the circuit board 1182 of the side rail 48.

Referring to Figs. 29-33, the light strip comprises an electrical substrate encapsulated in a transparent material. The light strip 1604 includes six blue LEDs 1610 positioned on the substrate in which alternate with six amber LEDs 1612. The end 1608 includes a stiffener 1614 which is provided for support for a connector 1616. The connector has alternate leads 1618, 1620, 1622, and 1624 with the leads 1620, 1624 providing a common to the respective LEDs 1610, 1612. The lead 1618 provides a current to the LEDs 1610 from the circuit board 1182 when the LEDs 1610 are to be illuminated. Similarly, the lead 1620 provides current to the LEDs 1612 when the LEDs 1612 are to be illuminated. A body 1626 of the light strip 1604 has a larger thickness and is relatively stiff. An adhesive backing 1628 is used to secure the light strip in the channel 1600. A tail 1630 is secured to the body 1626 but has sufficient flexibility to be routed through the side rail body 1136. As indicated in Fig. 33, a signal from the circuit board 1182 simultaneously illuminates all of the LEDs 1610.

In operation the light strip 1604 has three states, none of the LEDs 1610, 1612 being illuminated, the blue LEDs 1610 being illuminated, or the amber LEDs 1612 being illuminated. In the current embodiment, none of the LEDs 1610, 1612 are illuminated in one of two conditions: if the patient position monitoring system is disarmed and the patient is in hospital bed 10, or if the patient position monitoring system is armed and the patient is in the proper position. The blue LEDs 1610 are illuminated if the patient position monitoring system is disarmed the patient is out of the hospital bed 10. The blue LEDs 1610 tend to provide additional lighting for the patient if the ambient light is relatively low. The amber LEDs 1612 are illuminated if the patient position monitoring system is armed and the patient is not in the proper position. This amber illumination provides an additional indication to a caregiver of the alarm condition of the patient position monitoring system.

The notification system 796 is configurable to allow or prevent the illumination capabilities of the grip 1166. A caregiver may choose to disable the illuminated grips as a part of the notification system 796 when the caregiver determines that the operation of the illuminated grip 1166 is unnecessary or would be problematic with a particular patient. Thus, the caregiver can configure the notification system 796 to monitor one or more conditions and provide an indication to a caregiver by illuminating an indicator on the foot deck 34, projecting an image on the floor, and/or illuminating the grip 1166. In some embodiments, the illumination of the grip 1166 and the amber color may be configured to be based on a different condition, such as the expiration of a time between vital signs checks, or any other condition of which the caregiver might need to be reminded. In addition, the illuminated grip may be illuminated in the amber color if any of the alarm conditions of the hospital bed 10 are active, the amber color providing an indication to the caregiver then alarm condition, or a condition that does not meet a patient's care protocol exists.

Referring again now to Fig. 6, management of the cable 798 is accomplished with a rigid wire routing bracket 840 which is secured to the channel member 786 with a pair of fasteners 842 and extends from the channel member 786 through in opening 844 formed in a plate 846 of the frame 746 of the first portion 36. The cable 798 is secured to the rigid guide 840 by wire ties (not shown). A flexible guide 848 is secured to an end of the rigid guide 840 and secured to the first portion 36 by a bracket 852 which is secured to the first portion 36 with a fastener 854. The flexible guide 848 is constructed of a material that is flexible but has a sufficient cross-section to control the collapsing of the flexible guide 848 into a shape as shown in Fig. 6. The cable 798 is also secured with wire ties to the flexible guide 848 such that when the second portion 38 is retracted relative to the first portion 36, the flexible guide 848 controls gathering of the cable 798 within the footprint of the first portion 36. The combination of the rigid guide 840 and flexible guide 848 allows for controlled gathering of the cable 798 throughout the range of motion of the second portion 38 relative to the first portion 36 while preventing the cable 798 from drooping below the confines of the first portion 36.

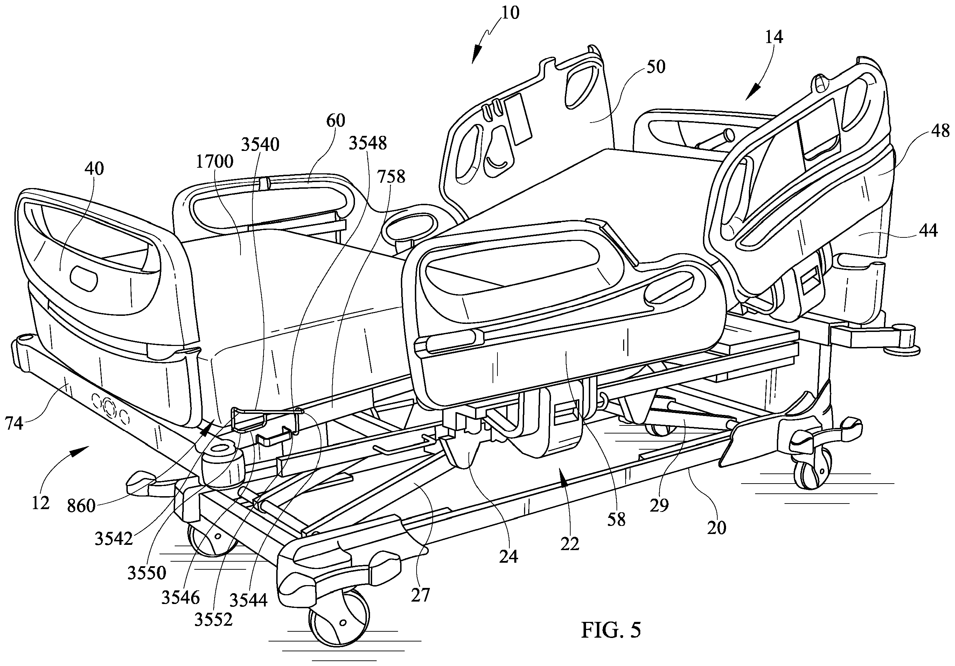

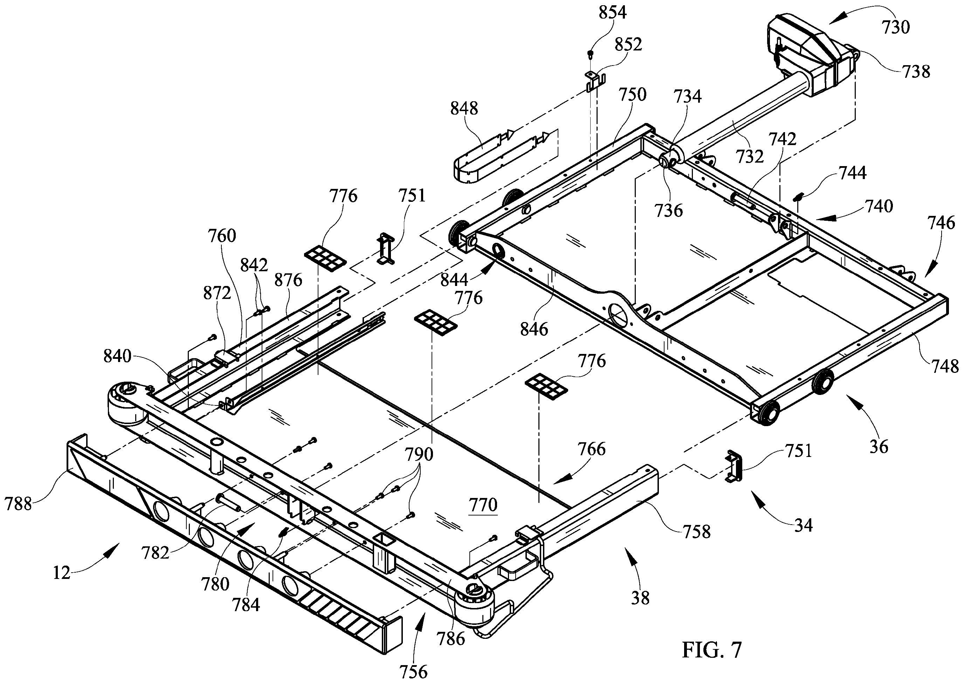

Referring again now to Fig. 7, the foot deck 34 includes a pair of wire form bag supports 860 and 862 with the bag supports 860 and 862 being symmetrical mirror images of each other. With reference to the bag support 860 it can be seen that the bag support 860 includes a first leg 864 which is linear and a second leg 866 which terminates in a hook 868. The leg 864 is positioned at a hole 870 formed in the channel member 786 and the hook is received in a bracket 872 seen in Fig. 6. The bag support 860 is positioned on the second portion 38 by inserting the leg 864 into the hole 870 and into a second hole 874 positioned on a lower flanges of the channel member 786. Once secured, the second leg 866 is deflected to permit the hook 868 to be positioned between the bracket 872 and a surface 876 of the channel member 760. Once the deflection of the leg 866 is released, the hook 868 engages the bracket 872 to secure the bag support 860 in place on the foot deck 34. When the bag supports 860, 862 are mounted to the second portion 38 of the foot deck 34 and move with the foot deck 34 as it is moved to various orientations relative to horizontal. Referring to Fig. 5, bag support 860 includes an upper rail 3540 that is not parallel to the rail 758 of the second portion 38. A first end 3542 is spaced apart from the rail 758 than a second end 3544.

The bag support 862 is positioned on the opposite side of the second portion 38 in a similar manner. The second portion 38 also supports a pair of bumpers 880 and 882 that are positioned at the corners of the foot deck 34 being received between flanges of the channel member 786. The bumpers 880 and 882 rotate on axles 884, 884 which are positioned on the channel member 786 with a slot 886 formed in each axle 884 engaging an anti-rotation feature 890 formed in the lower flange of the channel member 786. The axles 884, 884 are secured in place by retaining clips 892 to prevent rotation of the axles 884, 884 relative to the channel member 786. However, the bumpers 880, 882 are free to rotate about the axles 884 if they should come in contact with an outer surface, such as a wall, as the hospital bed 10 is moved.

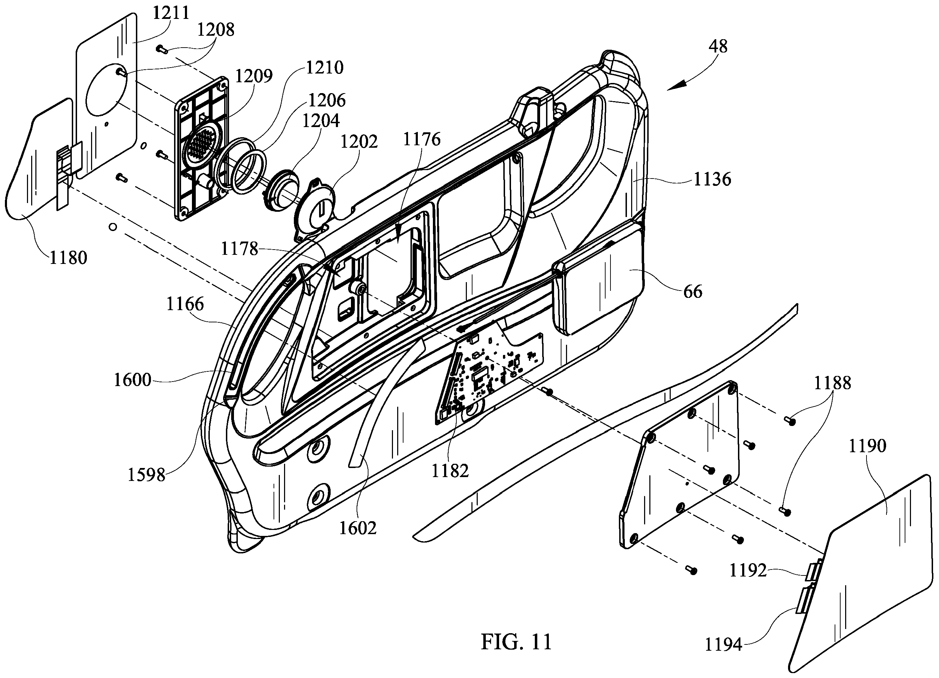

Fixed electronic controls accessible to a patient are positioned in a depression 1174 formed in the inboard side of the bodies 1130, 1136 and which communicates through the body 1130, 1136 through an opening 1176 to a depression 1178 formed in the outboard side of the bodies 1130, 1136. As shown in Fig. 11, a circuit board 1182 is positioned in the depression 1178 and secured by a fastener 1184 of a cover 1186 overlies the circuit board 1182 and is secured in place by six fasteners 1188 which are screwed into the body 1130, or 1136. A control panel 1190 includes a number of membrane switches which may be activated by a caregiver to control functions of the hospital bed 10. The functions controlled by the control panel 1190 will be discussed in further detail below. The control panel 1190 includes two flex circuits 1192, 1194 which connects to corresponding connectors 1196, 1198. The flex circuits 1192, 1194 are secured in place by the cover 1186 and the control panel 1190 is secured to the cover 1186 by an adhesive. The control panel 1190 is then covered by a label (not shown in Fig. 11) which will be discussed in further detail below, but which is positioned in the depression 1178 to seal the depression 1178.

A speaker assembly 1200 is positioned in the depression 1174 and the inboard side of the bodies 1130, 1136. The speaker assembly 1200 includes a speaker back 1202, a speaker 1204, and a foam ring seal 1206. A speaker cover 1209 is positioned in the depression 1174 and secured by four fasteners 1209. A second foam ring seal 1210 is positioned to prevent ingress of fluid from the speaker opening 1212 of the speaker cover 1209. The speaker cover 1209 is formed to include a receiver 1214 into which a USB charging receptacle 1216 is positioned. The USB charging receptacle 1216 provides appropriate electrical power and an outlet for a patient to plug a USB cable into to charge a device, such as a smart phone, for example. An overlay 1211 is positioned on the cover 1209 to provide a smooth surface and overlay the screws 1208.

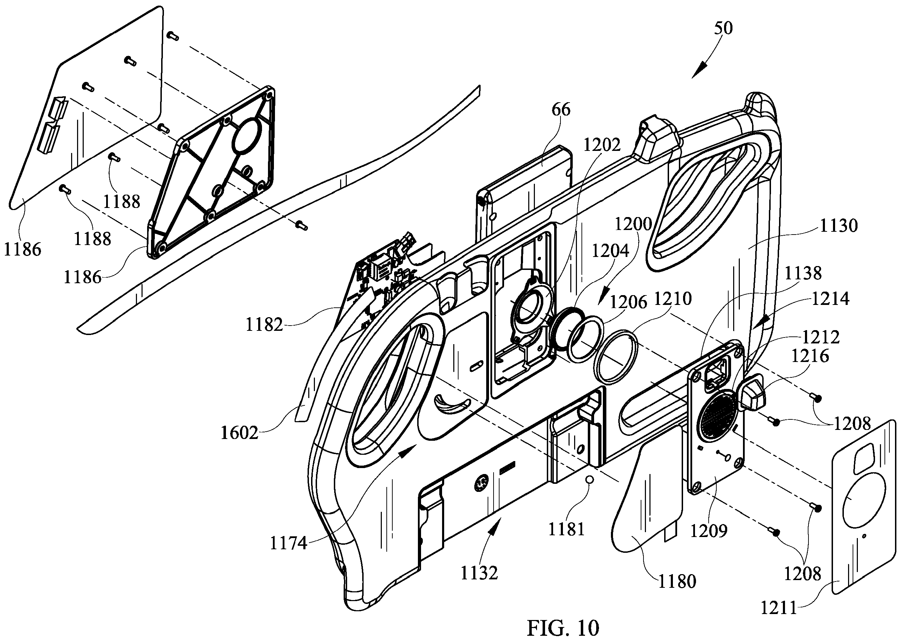

Referring to Figs. 10-11, each head side rail 48, 50 includes a cable guide 1230 positioned in the cavity 1132 and configured to manage a cable which connects the electronics of the side rails 48, 50 to the control system as will be described in further detail below. As shown in foot side rails 58 and 60, both of the side rails 58 and 60 have a similar construction, but are mirror images of each other. Each has a respective body 1232 and 1234. In the following discussion, the features of the bodies 1232, 1234 utilizing a single reference number for each feature with the understanding that the features are actually mirror images. The features that are present on the inboard side of the bodies 1232, 1234 will be discussed with reference to body 1234 and the features that are on the outboard side of the bodies 1232, 1234 will be discussed with reference to body 1232. Each of the bodies 1232, 1234 have a cavity 1132 configured as the cavities 1132, 1132 of bodies 1130, 1136 of the head rails 48, 50 and configured to receive a linkage 1134.

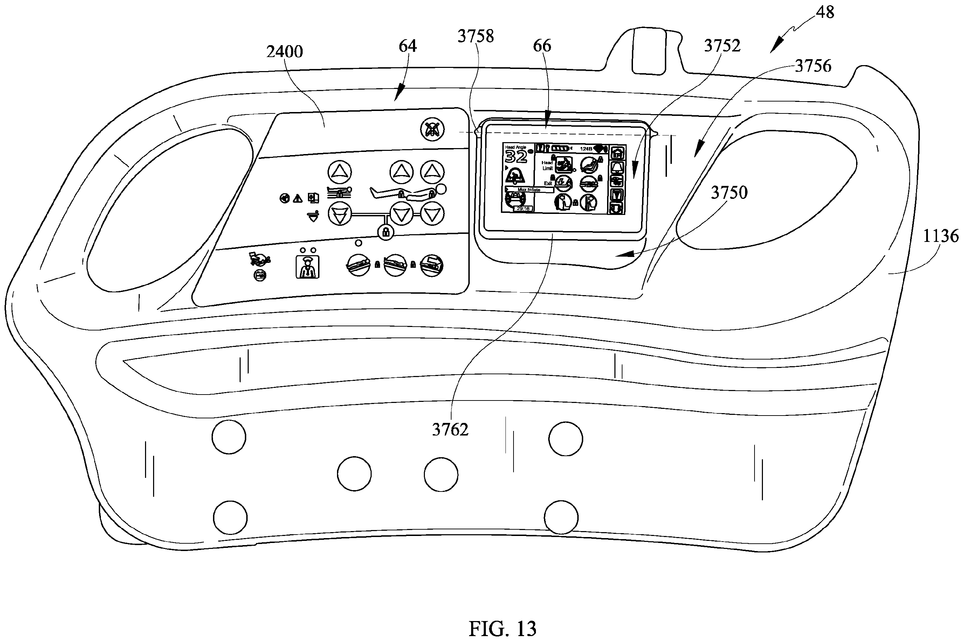

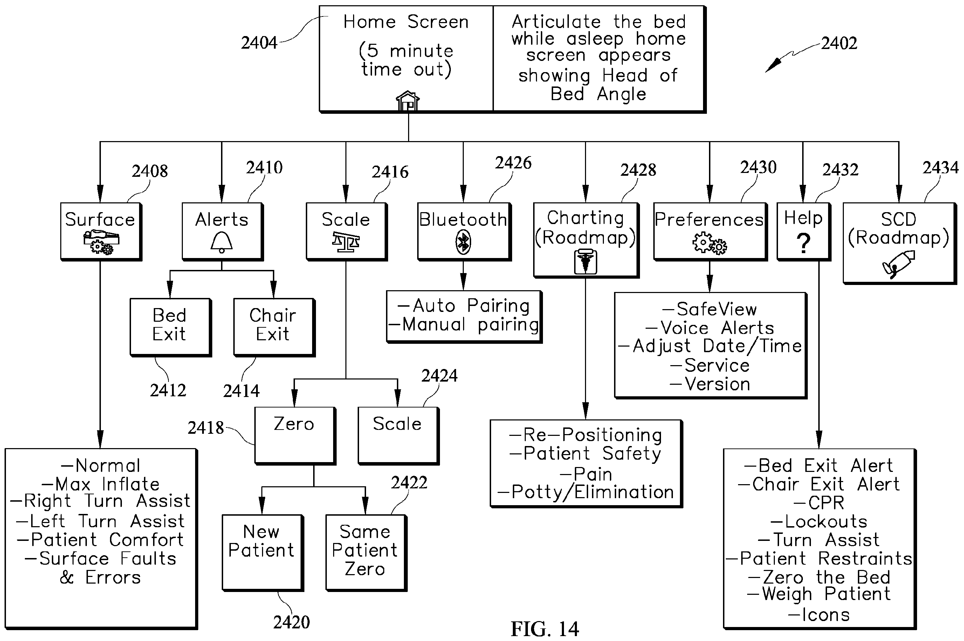

Referring to Fig. 13, in one embodiment the hard panel 64 includes a membrane switch assembly 2400 that provides access to a number of standard functions of the hospital bed 10 for a caregiver. The graphical user interface 66 is shown to have a number of iconic symbols which provide information to the caregiver and operate as soft keys for the caregiver to activate functions of the hospital bed 10. A high-level menu structure 2402 for the graphical user interface 66 is shown in Fig. 14. Under normal operating conditions, the graphical user interface 66 will display a home screen 2404 that is subject to a five-minute timeout which results in the home screen 2404 being replaced by a sleep screen 2406. The menu driven controls include a set of surface controls 2408 which allow a user to interact with the controls for the mattress 1900. And alerts structure 2410 allows the user to interface with patient position monitoring functionality 2412 or chair exiting functionality 2414. A scale structure 2416 allows a caregiver to access the operation of the scale system to utilize a zeroing function 2418 including the ability to zero the hospital bed 10 for a new patient under a structure 2420 or zero the hospital bed 10 for the same patient under menu structure 2422. In addition, the scale structure 2416 allows a user to access a weighing menu structure 2424. A Bluetooth^{®} menu structure 2426 allows a user to managing the pairing of devices with the Bluetooth^{®} functionality of the hospital bed 10. A charting menu structure 2428 provides a menu structure for a caregiver to chart information available from the control system to external networks connected to the hospital bed 10. The menu structure 2402 includes a menu structure 2430 which allows a caregiver to adjust various preferences relative to the graphical user interface 66 and hospital bed 10. Menu structure 2432 is available for a caregiver to understand the operation of the graphical user interface 66 and hospital bed 10. And a menu structure 2434 allows a user to adjust operations of a sequential compression device when such a device is attached to the hospital bed 10.

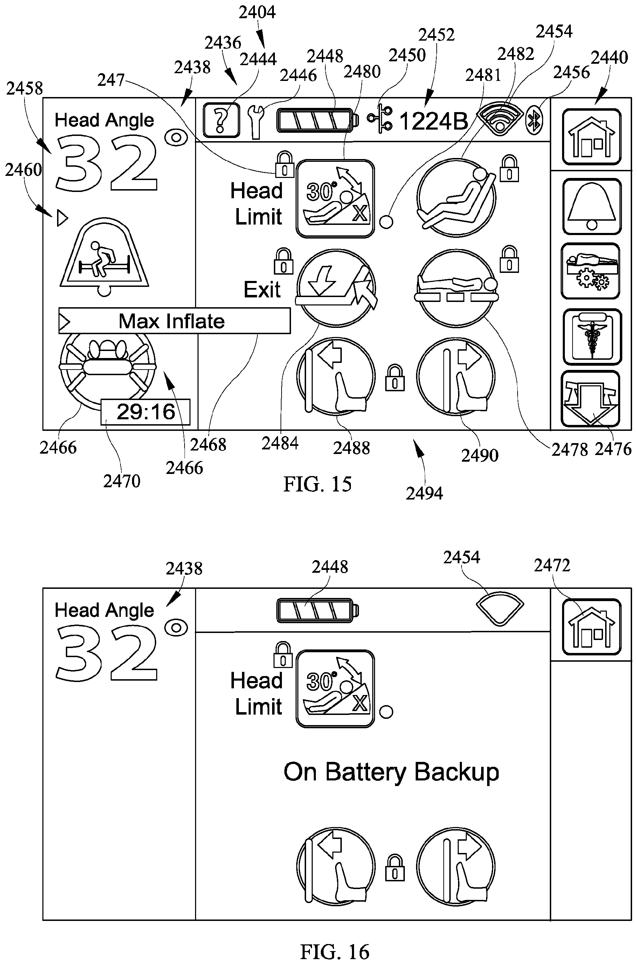

The home screen 2404 is shown in detail in Fig. 15 and includes an information section 2436, a status section 2438, a menu section 2440, and an interaction section 2442. The information section 2436 includes a help screen icon 2444 which activates the help screen when touched by user. In addition a maintenance indicator 2446 provides an indication that the hospital bed 10 requires maintenance. A battery status indicator 2448 displays a graphical representation of the charging status of a battery for the hospital bed 10. A network indicator 2450 is illuminated when the hospital bed 10 is connected to an external network, such as a nurse call network; including the NaviCare^{®} nurse call system available from Hill-Rom, for example. When the hospital bed 10 is connected to a network that includes location information, the room number or other location identifying information is displayed on the information section 2436 as indicated by reference numeral 2452. An icon 2454, when present, provides an indication that the hospital bed 10 is connected to a Wi-Fi system. Similarly, an icon 2456, when present, provides an indication of the hospital bed 10 is connected to another device via a Bluetooth^{®} connection.

A status section 2438 includes an indicator 2458 which provides a display of the current head angle of the hospital bed 10. A location 2460 of the status section 2438 provides an indication that that the hospital bed 10 is monitoring for an alert condition, such as an alert condition assisted with a patient position monitoring system. For example, the icon 2462 shown in Fig. 15 provides an indication that the patient position monitoring system is set to alert if the patient exits the hospital bed 10. A third portion 2464 of the status section 2438 provides the indication of the status of a subsystem. An icon 2466 provides an iconic representation of the status of an optional pneumatic mattress being in a maximum inflate mode. The icon 2466 may have components that flash, blank, illuminated in sequence, or otherwise provide an animated indication that a status is active. In addition, a text box 2468 is displayed to indicate the condition in a text form. In the case of the maximum inflate mode, a second text box 2470 displays a timer indicating the amount of time that the system will permit the mattress 1900 to be maintained in the current state. In some embodiments, the text box 2468 is omitted and only an icon, such as the icon 2466 is displayed. The text box 2470 may not be present if there is no limit on the time for the mattress 1900 to be in the current condition. While the status section 2438 in the illustrative embodiment of Fig. 15 displays information regarding alerts at the location 2460 and a status of an optional pneumatic mattress at the location 2464, in other embodiments the status of other subsystems may be displayed within the status section of the home screen 2404.

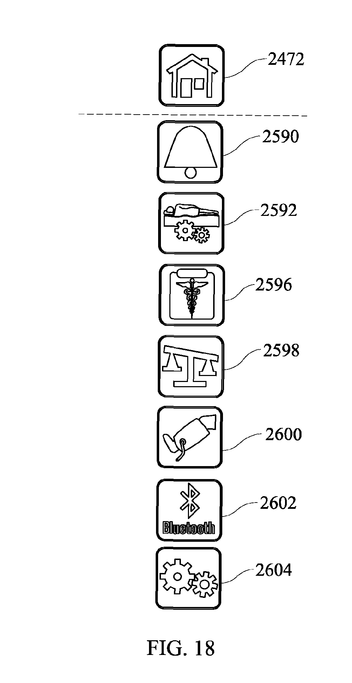

The menu section 2440 of the home screen 2404 includes a home screen icon 2472 which is generally always present on the display of the graphical user interface 66. When the home screen icon 2472 is activated by a caregiver, the home screen 2404 is displayed. A section 2474 of the menu section 2440 includes a number of icons which may be scrolled through by activating an arrow icon 2476 positioned at the bottom of the section 2474. The icons of the section 2474 are shown in Fig 18 in the order that they appear in the section 2474. An alerts icon 2590, when activated, causes the alerts menu structure 2410 to become active in the interaction section 2442. A surface icon 2592, when activated, causes the menu structure 2408 to become active in the interaction section 2442. Activation of a charting icon 2596 causes the charting menu structure 2428 to become active in the interaction section 2442. Activation of the scale icon 2598 causes the scale menu structure 2416 to become active in the interaction section 2442. The SCD icon 2600 is associated with the SCD menu structure 2434. The Bluetooth^{®} icon 2602 causes the Bluetooth^{®} menu structure 2426 to be displayed in the interaction section 2442. Activation of preferences icon 2604 causes the preferences menu structure 2430 to become active in the interaction section 2442.

The interaction section 2442 displays up to six functions which may be activated by a caregiver from the home screen 2404. An icon 2480 is associated with a head angle limit alert and when activated will cause a warning to be displayed if the angle of the head deck 28 relative to the relative to the load frame 26 is lowered below 30°. This function may be activated if the patient has a risk factor that requires the patient's upper body to be maintained in an upright position. When the head limit is activated an indicator 2481 adjacent the icon 2480 is illuminated. In some cases, modification of the head limit may be restricted. The operation of the hospital bed 10 may be adjusted so that activation and deactivation of the head limit by icon 2480 is locked out so that an inadvertent activation of the icon 2480 does not toggle the alert monitoring to an off position. When a function, such as the head limit the function, is locked out, a lockout indicator 2478 is displayed adjacent the icon for the particular function.

An icon 2482 may be activated by a caregiver to cause automatic movement of the head deck 28, articulated seat deck 30, and foot deck 34 to a chair position, such as the position shown in Fig. 5. Activation of the icon 2482 may also cause the lift system to operate such that the foot end 12 of the load frame is lowered relative to the head end 14. Activation of the icon 2484 will cause the head deck 28 to be raised with the remainder of the hospital bed 10 placed in a flat condition to ease the exiting of the hospital bed 10 by a patient. In some embodiments, activation of the icon 2484 may also affect the operation of the mattress 1900 when it is present. For example, activation of the icon 2484 may cause the body support 1902 to be inflated to a pressure higher than normal to cause the body support 1902 to be stiffer and improve the support of the patient's buttocks as they are exiting the hospital bed 10. Activation of the icon 2486 will cause the head deck 28, articulated seat deck 30, and foot deck 34 to be placed in a flat condition while also causing the lift system 22 to be moved to cause the load frame 26 to be in a horizontal position. The interaction section 2442 also displays a foot retraction control section 2494 which includes an icon 2488 which may be activated to cause the foot deck 34 to be extended and an icon 2490 which may be activated to cause the foot deck 34 to be retracted. Some of the icons displayed in the interaction section 2442 of the home screen 2404 may not be present if the associated functionality is omitted from the hospital bed 10. For example, some embodiments of hospital bed 10 do not include a powered foot deck 34, and therefore the foot retraction control section 2494 would not be present in those embodiments.

When the hospital bed 10 is disconnected from a mains power source, the hospital bed 10 may be operated by the batteries. When the hospital bed 10 is on battery power, the interaction section 2442 displays the text "On Battery Backup" in the center of the interaction section 2442. The head limit icon 2480 and associated indicator 2481 are also displayed as that function remains active. In addition, the foot control retraction section 2294 remains displayed because that function is also available under battery backup. The home screen icon 2472 remains visible such that a caregiver is allowed to activate the home screen 2404. However, the home screen 2404 will revert to the battery backup screen 2492 after a period of time of no interactions with the home screen 2404, such as a time period of 30 seconds, for example. The other functions that appear on the home screen 2404 are not displayed when the hospital bed 10 is on battery backup as those functions are not available under battery power. Any motion of any portion of the hospital bed 10 has to be engaged individually by the keys on the hard panel 64.

In some embodiments, if any of the icons 2480, 2482, 2484, 2486, 2488, or 2496 are activated, animated arrows or other indicators may appear within the icon to indicate that the function is being activated.

Referring now to Fig. 13, the side rail 48 is shown with the graphical user interface 66 positioned in a cavity 3750. The graphical user interface has a surface 3752 on the front of the cover 3754, which is generally flush with the surface 3756 of the body 1136 of the side rail when the graphical user interface 66 is stowed. The graphical user interface 66 is pivotable about an axis 3758 if it is gripped by a user at the bottom 3762 and lifted upwardly about a pivoting structure that will be described in further detail below. The axis 3758 is defined by an opening 3760 shown in Fig. 28, the opening 3760 being formed in a wall in the cavity 3750. A second opening, not visible, is aligned with opening 3760 on the opposite side of cavity 3750.

The graphical user interface 66 may be positioned in a cavity of side rail 50 that is a mirror image to the cavity 3750. Because of the mirror image aspect, the graphical user interface 66 interfaces with the circuit board 1182 on its left head rail 48, but the circuit board 1182 is to the right of the graphical user interface 66 on the right head rail 50.

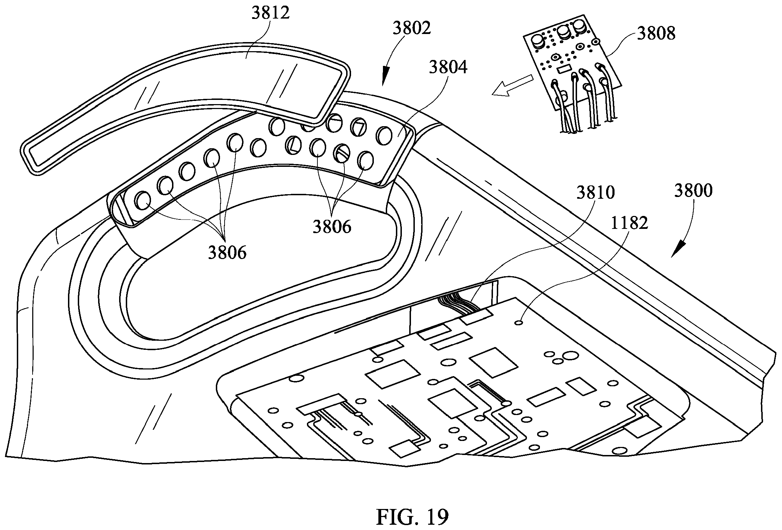

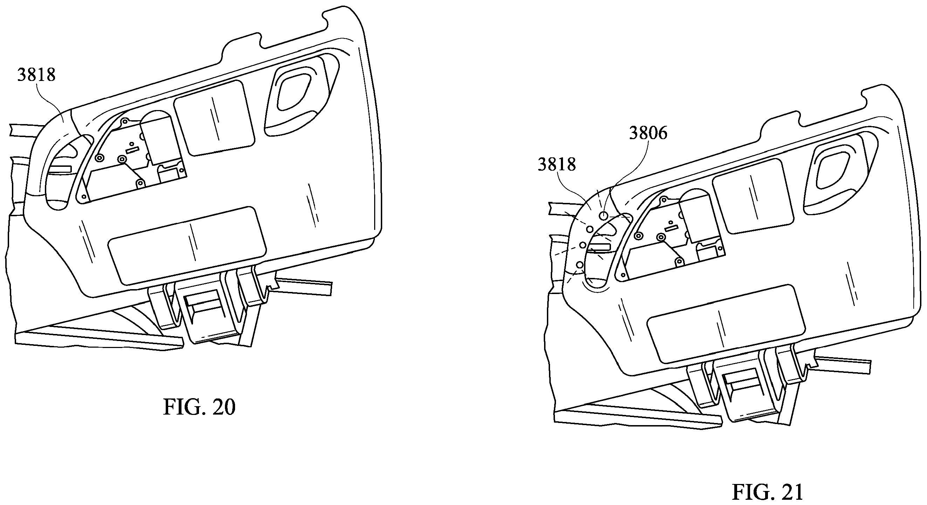

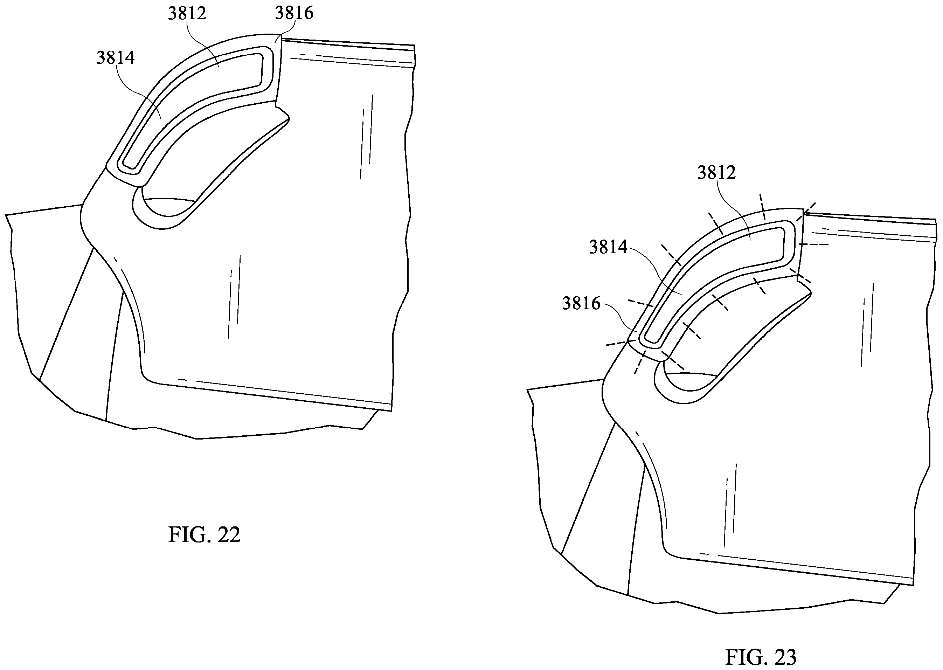

Referring now to Fig. 19, another embodiment of a side rail 3800 is configured to have an illuminated grip 3802 includes a depression 3804 formed on the outer side of the grip 3802. A number of holes 3806 are formed in the grip at the depression a circuit board assembly 3808 which includes a number of different color LEDs that operate under the same logic as discussed above with regard to the notification system 796. The circuit board assembly 3808 is connected to the circuit board 1182 by a wire harness 3810. The translucent overlay 3812 is positioned into the depression 3804 to thereby fill the depression 3805 and provide a smooth surface at the grip 3802. The overlay 3812 has an opaque region 3814 with a translucent area 3816 about the opaque section 3814. As suggested by Fig. 23 the light emitted by the diodes on the circuit board 3808 emit from the translucent area providing a subdued effect. In another embodiment shown in Fig. 20 an overlay 3818 is a solid translucent material which permits the holes 3806 to appear much more clearly when the LEDs illuminate. In some environments a brighter illumination such as that suggested by the overlay 3818 may be appropriate. In other instances, the overlay 3812 may be more appropriate to provide the subdued lighting effect.

In general, the control system could be arranged in many different configurations, but the contemplated embodiments would employ a mix of network communications protocols depending on the functionality required. The communication circuitry may be configured to use any one or more communication technology (e.g., wired or wireless communications) and associated protocols (e.g., Ethernet, Bluetooth^{®®}, Wi-Fi^{®}, WiMAX, etc.) to effect such communication.

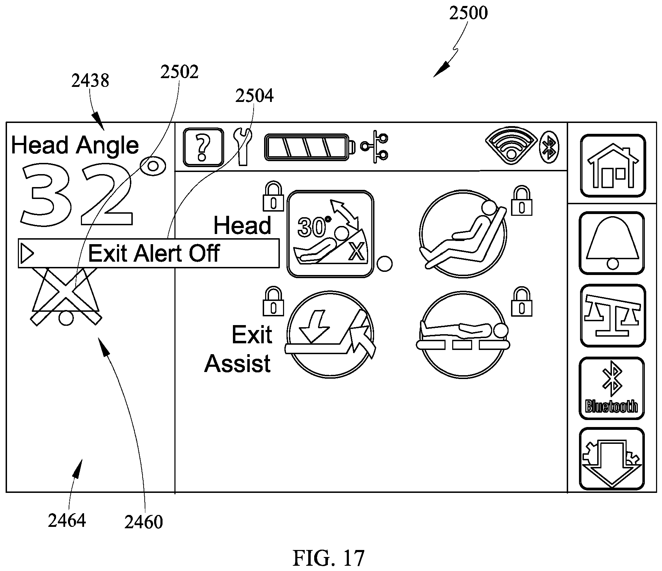

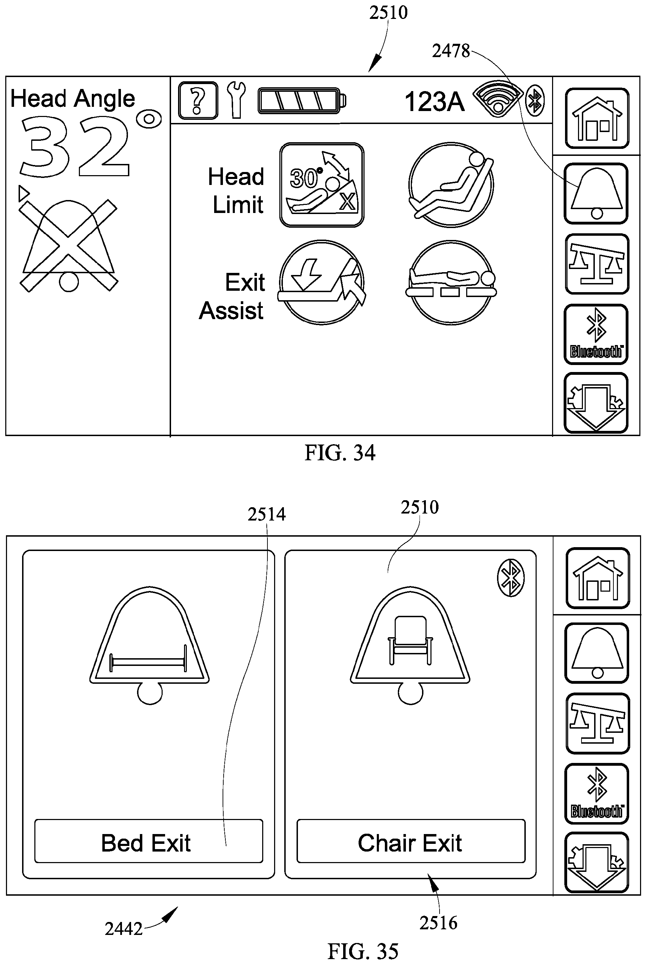

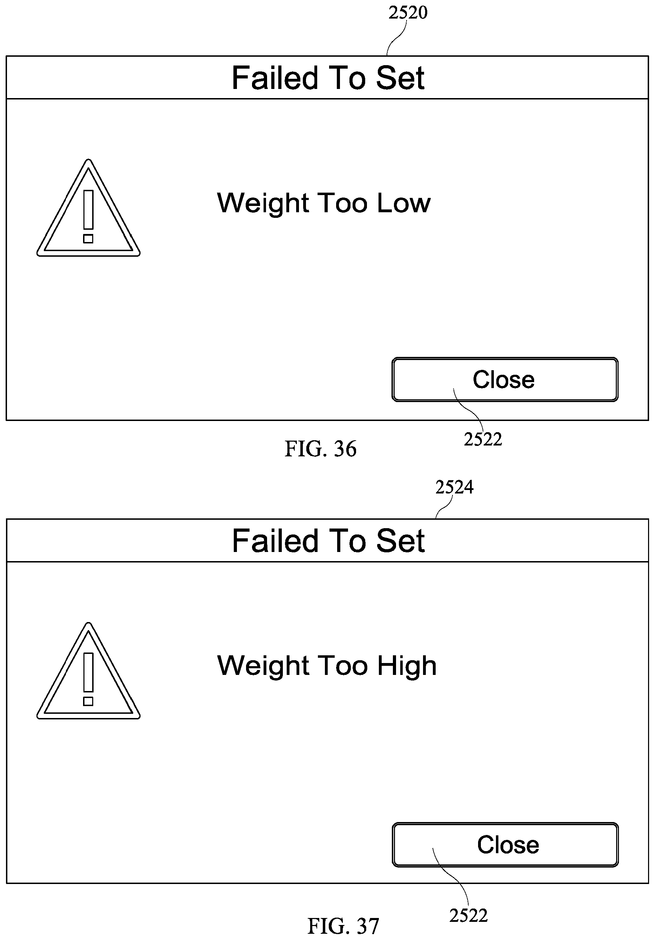

In another embodiment of a screen 2500 shown in Fig. 17, the portion 2464 of status section 2438 does not provide any indication when the mattress 1900 is absent as there is no functionality available. Similarly the foot control retraction section 2294 is blank when the actuator 730 is absent as there is no powered extension and retraction of foot deck 34. In the embodiment of screen 2500, the section 2460 displays an icon 2502 which provides an indication that patient position monitoring system is inactive with a text box 2504 providing text explaining the status of the alerts for the patient position monitoring system. The text box 2504 and text box 2468 of Fig. 15 are temporarily displayed but disappear after a period of time, such as five seconds, for example. In the display shown by a screen 2510 of Fig. 34, the alerts icon 2590 is shown to be activated which invokes the alerts menu structure 2410. Figs 34-70 show the various screens of the alert menu structure 2410 with a screen 2512 being displayed upon activation of the alert icon 2590. The screen 2512 includes an expanded interaction section 2442 which expands to overlie the information section 2436 and the status section 2438. Screen 2512 displays two options including a virtual button 2514 that is associated with a hospital bed 10 exit alert menu structure and a virtual button 2516 associated with a chair exit menu structure as shown in Fig. 35.

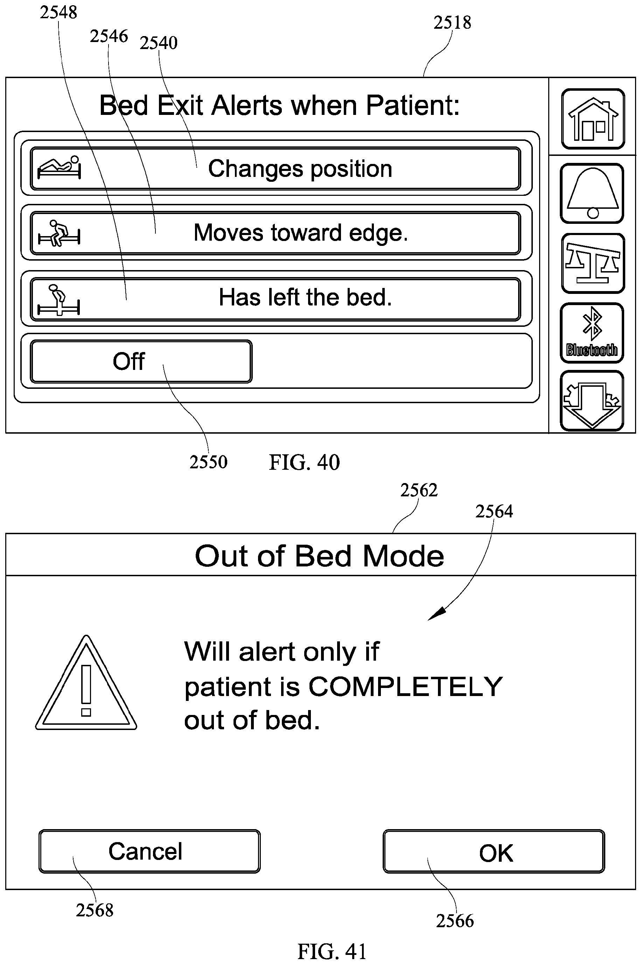

When the virtual button 2514 is activated, the menu structure advances to a screen 2518 shown in Fig. 40. However, if the weight supported on the hospital bed 10 is too low, a screen 2520 is displayed with the text indication that the alert system failed to set because the weight was too low. The caregiver has to activate a virtual button 2522 to return to the home screen 2404. If the virtual button 2522 is not activated, the screen 2520 will timeout and return to the home screen 2404 after a period of time, such as two minutes, for example. The hospital bed 10 exit alert will not set if the weight on the hospital bed 10 is too high and a screen 2524 will be displayed with text indicating that the system failed to set because the weight was too high while displaying the virtual button 2522 which allows the caregiver to return to the home screen 2404. The screen 2524 will also timeout, in a manner similar to the screen 2520.

In some cases, if the hospital bed 10 is not in an appropriate position or the patient is not appropriately positioned on the hospital bed 10, the hospital bed 10 exit alert will not set. The control system provides an indication to a caregiver through the graphical user interface 66 with a screen 2526 providing text indicating that the hospital bed 10 exit alarm failed to set with a text prompt 2532 prompt suggesting that the caregiver attempt to level the hospital bed 10 and try to set the system again. The screen 2526 times out after a period of time or can be closed out by activating the virtual button 2522 displayed on screen 2526 to return to the home screen 2404. If the control system determines that the patient is not appropriately positioned on the hospital bed 10, a screen 2528 is displayed providing a notification that the hospital bed 10 exit alarm failed to set. Screen 2528 provides a text prompt 2530 instructing the caregiver to center the patient and then set the hospital bed 10 exit. The caregiver is given the option of activating a virtual button 2534 causes the system to return to the home screen 2404, or adjusting the patient in activating a virtual button 2536 to make another attempt to set the hospital bed 10 exit alert.

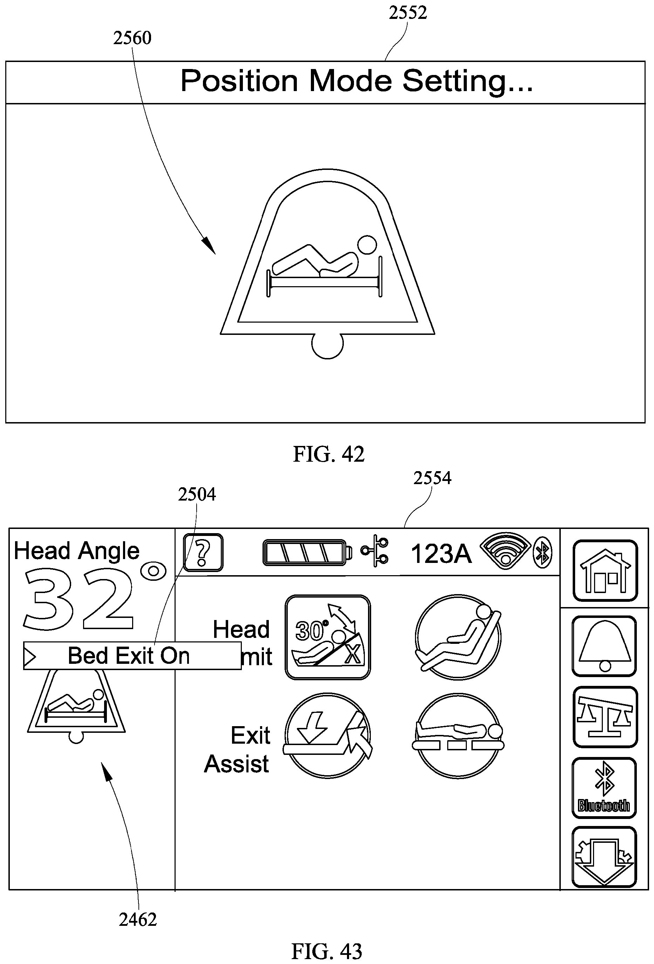

If no errors are detected, the screen 2518 is displayed and the caregivers given the option of choosing between three virtual buttons 2540, 2546, 2548 to set the hospital bed 10 exit alert in one of three modes, or a virtual button 2550 which turns off the hospital bed 10 exit alert system and returns the display to the home screen 2404. If the caregiver chooses the virtual button 2540, the hospital bed 10 exit alert is set to be sensitive to changes in the position of the patient and provide an alert if the patient does change position. The setting is the most sensitive of the three settings available in the hospital bed 10 exit alert menu structure 2412. Once the virtual button 2540 is selected screen 2552, which is shown in Fig. 42, is displayed to provide a text notification that the position mode is being set with a large version of a position mode icon 2560 being displayed while the hospital bed 10 exit alert system is set. Once the position mode is set, a screen 2554, shown in Fig. 43, is displayed with the icon 2560 being displayed in the status section 2438 and the text box 2504 temporarily providing a text prompt indicating that hospital bed 10 exit alerting has been set.

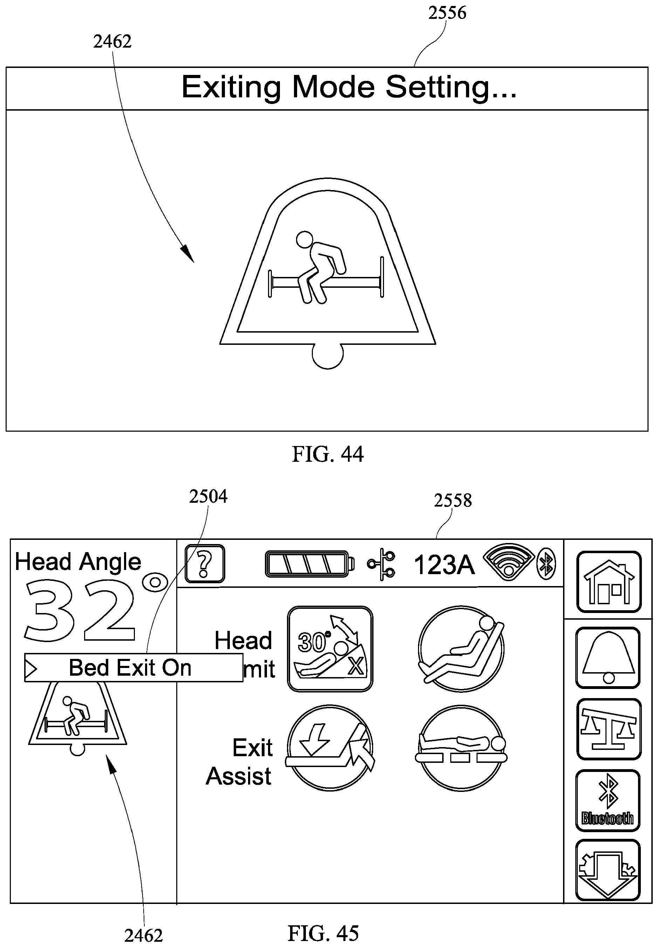

If the virtual button 2546 is activated, then a screen 2556, shown in Fig. 44, is displayed. The virtual button 2546 activates the exiting mode of the hospital bed 10 exit alerts. In this mode, the control system monitors to determine if the patient moves towards the edge of the hospital bed 10, indicating the patient intends to exit the hospital bed 10. If such a movement is determined to be occurring, the control system will provide an indication that the alert condition exists. While the exiting mode is being set, a large version of the icon 2462 is displayed on the screen 2556 with a text prompt in forming a user that the exiting mode is being set. Once the exiting mode is successfully set, the screen 2558 shown in Fig. 45 is displayed. On screen 2558, the text box 2504 provides the temporary indication that the hospital bed 10 exit alert system is active and the icon 2462 is displayed in the status section 2438 to provide an indication of the type of alert that is set.

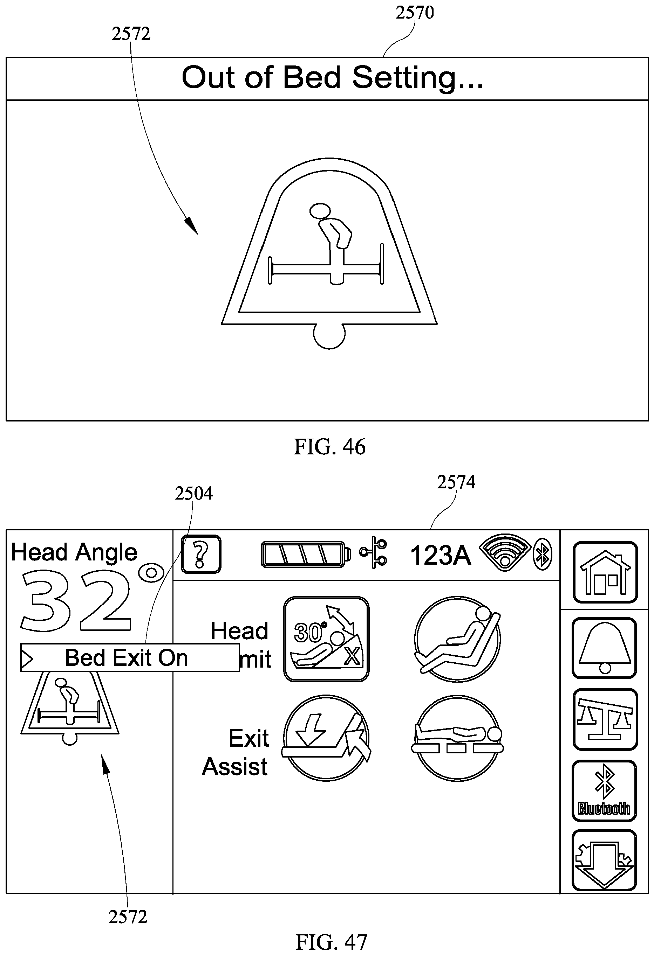

If the virtual button 2548 on screen 2518 is selected, then a screen 2562, shown in Fig. 41, providing a text message 2564 informing the user that the mode associated with virtual button 2548, the out of hospital bed mode, will only provide an alert if the patient is completely out of the hospital bed 10. The user must confirm that this is acceptable by activating a virtual button 2566 to allow the out of hospital bed alert to be set, or must select a virtual button 2568 canceling the out of hospital bed mode and returning to screen 2518. If the virtual button 2566 is activated, then a screen 2570, shown in Fig. 46, is displayed with a large version of an out of hospital bed 10 icon 2572 being displayed along with a text prompt in forming a user that the out of hospital bed alert setting is being set. Once the out of hospital bed alert setting is set, a screen 2574, shown in Fig. 47, is displayed with the out of hospital bed 10 icon 2572 being displayed in the status section 2438 and the text box 2502 being temporarily displayed.

If the virtual button 2516 associated with the setting of the chair exit alert menu structure 2414 is activated, a screen 2576, shown in Fig. 48, is displayed providing a user the opportunity to activate a virtual button 2578 or a virtual button 2580. The virtual button 2580 will cause the alert menu structure 2410 to be terminated and the home screen 2404 to be displayed. If the virtual button 2578 is activated, and a patient is properly positioned in a chair 2582, shown in Fig. 12.

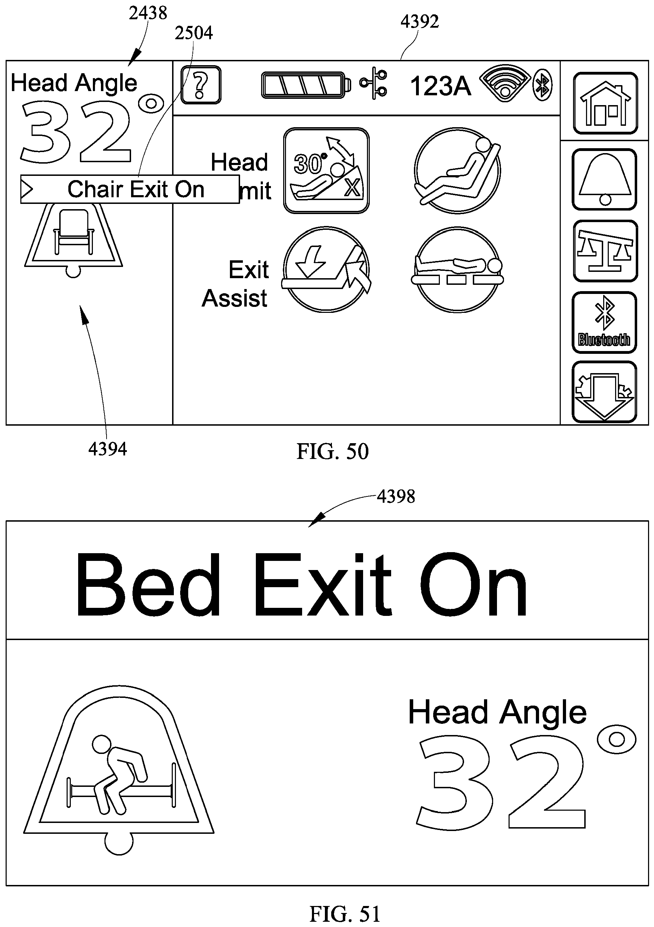

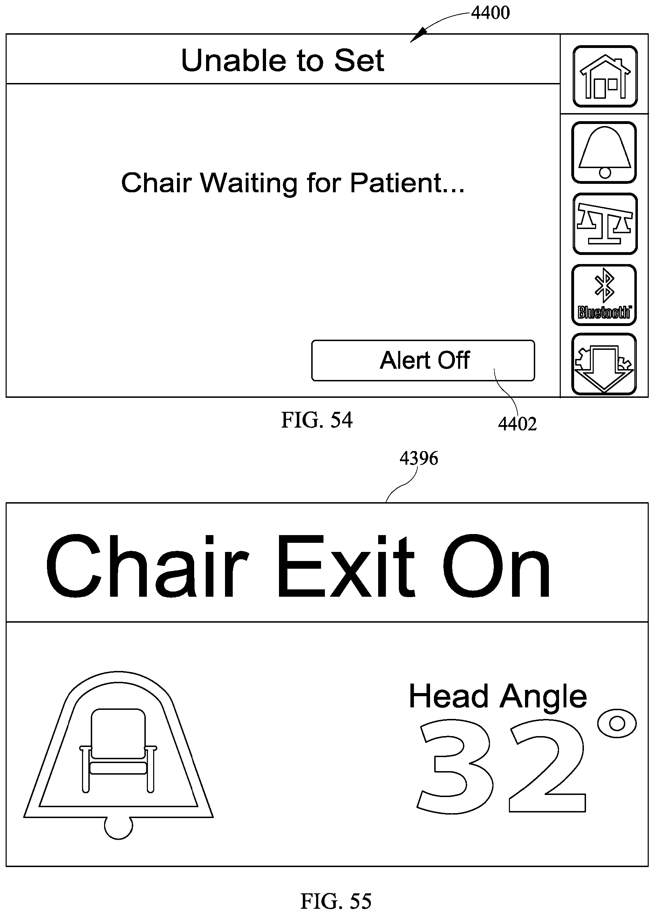

If the virtual button 2516 associated with the setting of the chair exit alert menu structure 2414 is activated, a screen 2576, shown in Fig. 48, is displayed providing a user the opportunity to activate a virtual button 2578 or a virtual button 2580. The virtual button 2580 will cause the alert menu structure 2410 to be terminated and the home screen 2404 to be displayed. If the virtual button 2578 is activated, and a patient is properly positioned in a chair 2582 shown in Fig. 12 then screen 4390 shown in Fig. 49 is displayed while the chair exit sets. If the chair exit alert effectively sets, then the menu advances to screen 4392 shown in Fig. 50 which is a home screen providing the status of the chair exit in the text box 2504 and displaying a chair exit alert active icon 4394 in the status section 2438. Once the home screen times out with the chair exit alert set, the menu advances to a screen 4396 shown in Fig. 55. Similarly, if the home screen shown in Fig. 45 times out, then the screen 4398 shown in Fig. 51 is displayed while the bed exit is active, including displaying the appropriate icon based on what the setting is for the alert.

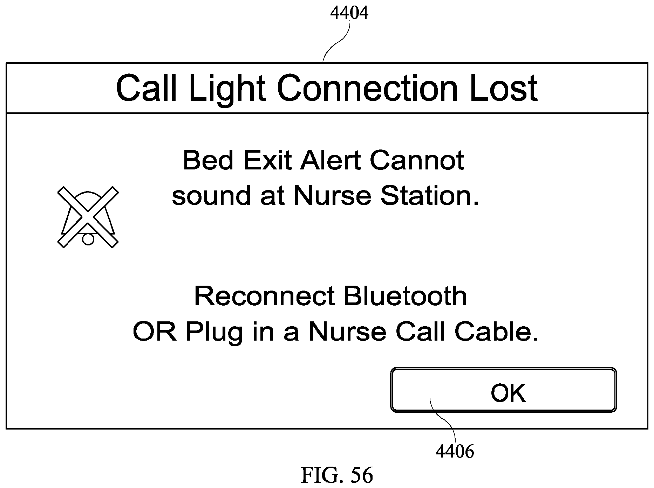

If the chair alert is set but there is no patient in the chair, the screen 4400 shown in Fig. 54 will be displayed. Screen 4400 gives a caregiver the opportunity to turn the alerts off by activating a virtual button 4402. The control system is also operable to let the caregiver know if the communication between the hospital bed 10 and another device or system is lost. For example, a screen 4404, shown in Fig. 56 is displayed if the nurse call cable or a Bluetooth^{®} connection is lost. A virtual button 4406 allows the caregiver to acknowledge the message and return to the home screen. The message does not timeout, but is displayed continuously until addressed. However, if the wired connection is lost, the control system will automatically connect via the wireless connection, Bluetooth^{®}, for example.

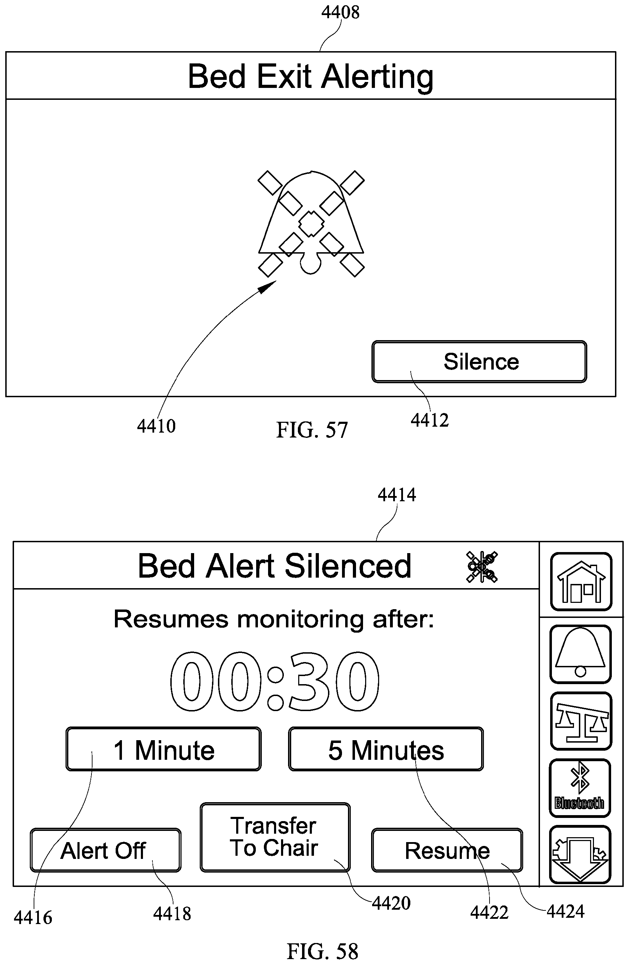

If a bed exit alert is triggered the screen 2408, shown in Fig. 57, will appear with an icon 4410 indicating that the alarm condition has been met. A virtual button 4412 allows the caregiver to silence the alarm. If the alarm is silenced in the patient is still on the bed, the menu structure advances to screen 4414 shown in Fig. 58. The monitoring system will return to monitoring within 30 seconds with a countdown timer showing the time to the restart of the alert. The caregiver can select from multiple virtual buttons with a virtual button 4416 extending the silenced alert for one minute. A virtual button 4418 may be activated to turn the alert off. A virtual button 4420 may be activated to commence with transferring the patient to a chair. A virtual button 4422 allows the silencing of the alert to be extended for five minutes. In a virtual button 4424 causes the alert to be resumed. It should be noted that the virtual button 4420 does not appear if the chair exit system is not available by Bluetooth^{®}.

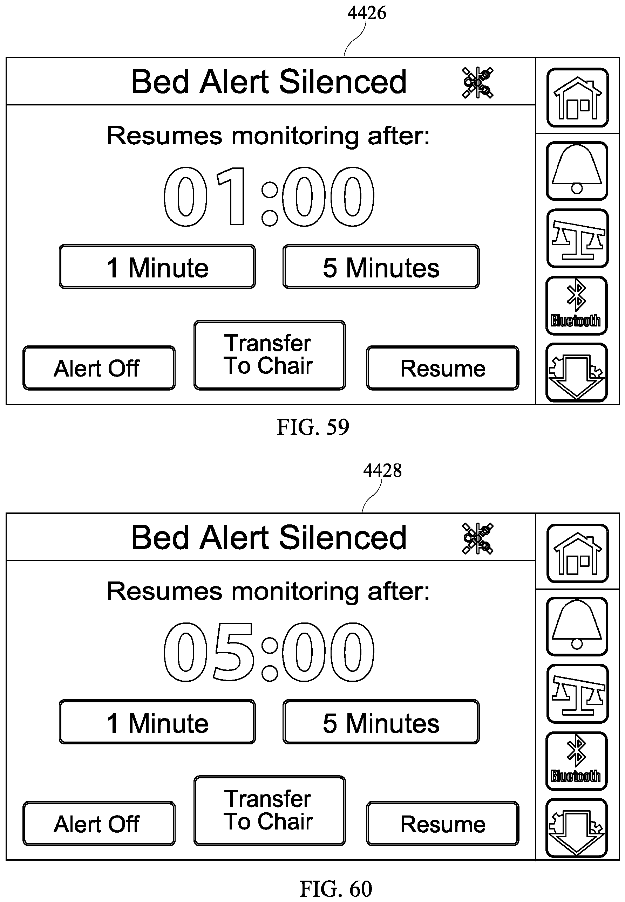

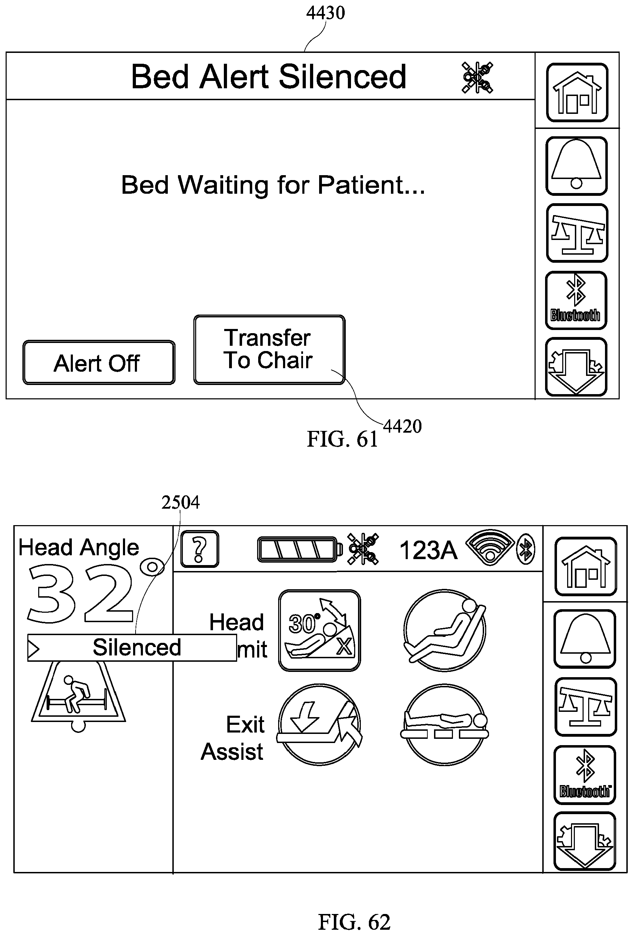

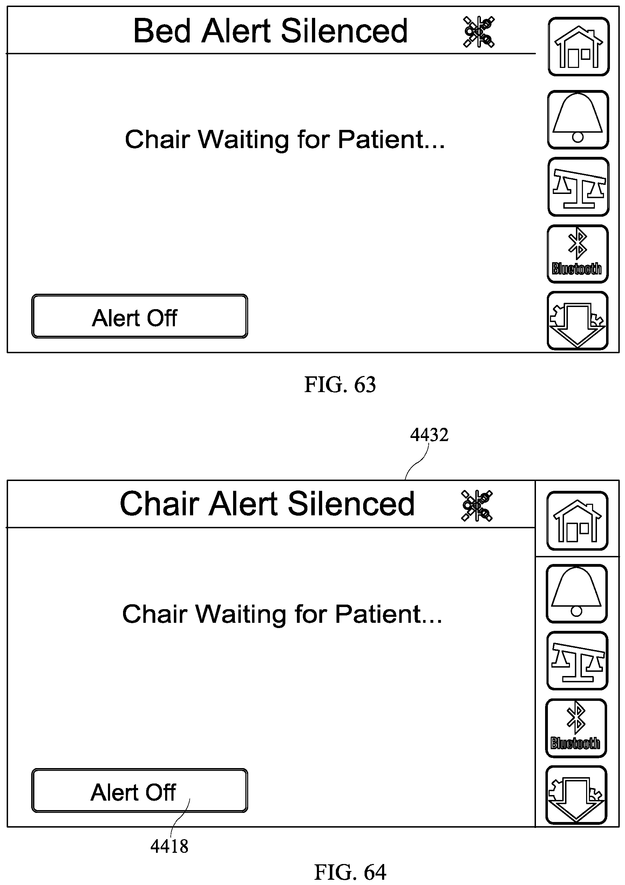

If virtual button 4416 is selected then the screen 4426 shown in Fig. 59 is displayed with the one minute countdown timer being active. If the five-minute virtual button 4422 is selected then screen 4428, shown in Fig. 60, is displayed. It should be noted that all of the virtual buttons 4416, 4418, 4420, 4422, 4424 are available in either screen 4426 or 4428. If the virtual button 4412 is selected at screen 4408, the menu structure advances directly to screen 4430 which prompts a caregiver that the bed is waiting for the patient to reenter the bed. Presumably the caregiver is aware of the patient's exit from the bed in his addressing the issue without turning the alerts off. The virtual button 4420 is available at screen 4430. If virtual button 4420 is selected at any time during a bed exit alert, the menu structure will advance to screen 4432 displayed in Fig. 64. Screen 4432 provides the prompt that the chair is waiting for the patient to be positioned in the chair. The alert off virtual button 4418 is available in screen 4432. If a caregiver attempts to navigate away from either screen 4434 or 4432 then the home screen shown in Fig. 62 will be displayed showing that the alarm is silenced in the text box 2504.

If the patient enters the chair while screen 4432 is displayed, and the menu structure will return to screen 2576 shown in Fig. 48.

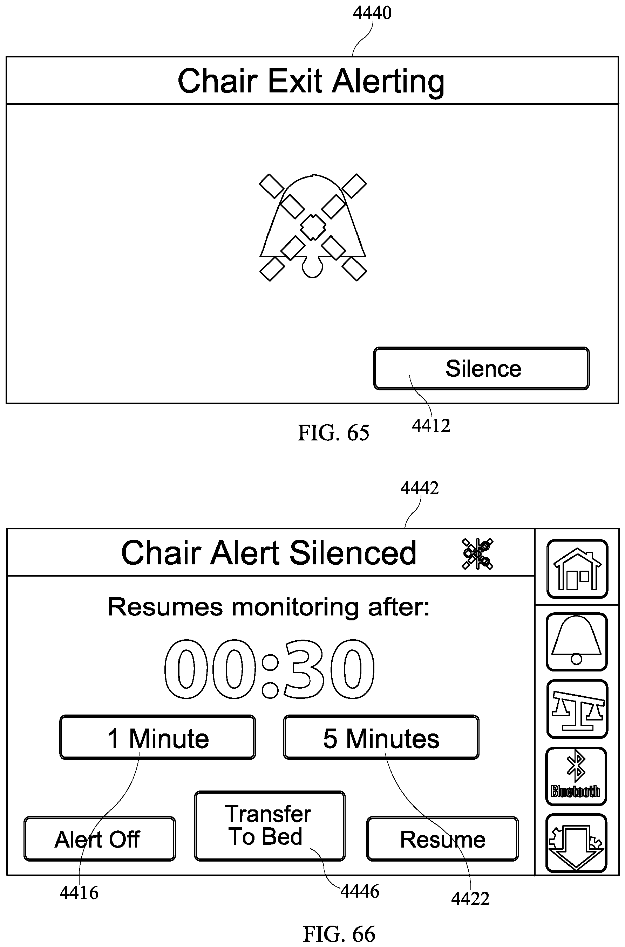

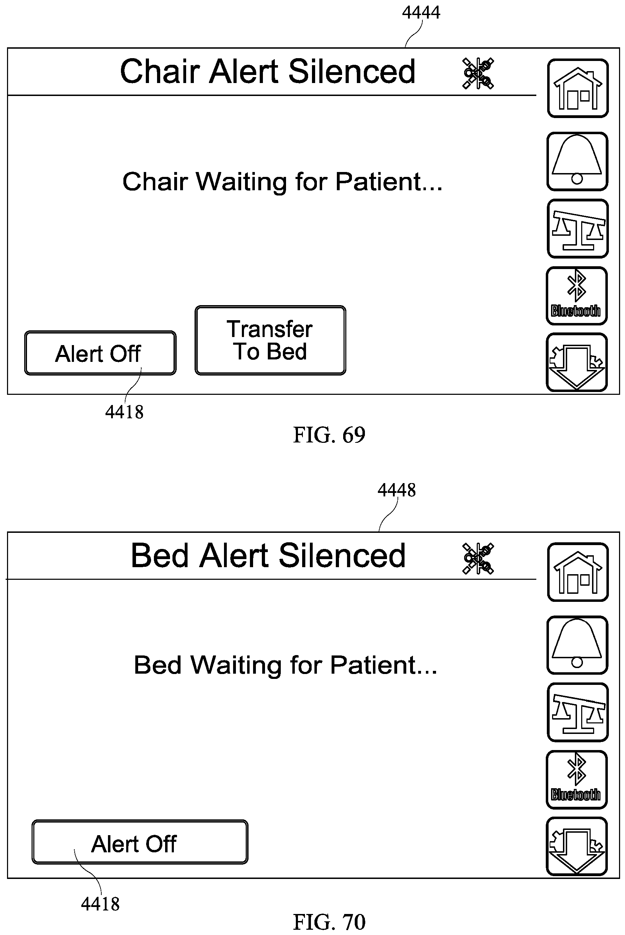

When chair exit alerting is active and a patient exits the chair, the screen 4440 shown in Fig. 65 will be displayed. The virtual buttons 4412 is available and if activated while the patient is in the chair the chair monitor resumes monitoring after 30 seconds as indicated by screen 4442 shown in Fig. 66 if the patient is not in the chair the menu structure advances to screen 4444 shown in Fig. 69 and the chair monitor waits for the patient to return to the chair. The caregiver can select either virtual button 4422 444 16 at screen 4442 to extend the alert silence. A virtual button 4446 also appears which, when activated allows the patient to be transferred to the bed which will result in the screen 4448 shown in Fig. 70 being displayed. The alert off virtual button 4418 is also available and in any case where the virtual button 4418 is activated, the system will return to the home screen. For clarification should be understood that screens 4450 or 4452 are only displayed when virtual button 4416 or virtual button 4422 are activated, respectively. If the patient returns to the bed while screen 4448 is active then the menu structure returns to the bed monitoring shown in Fig. 45.

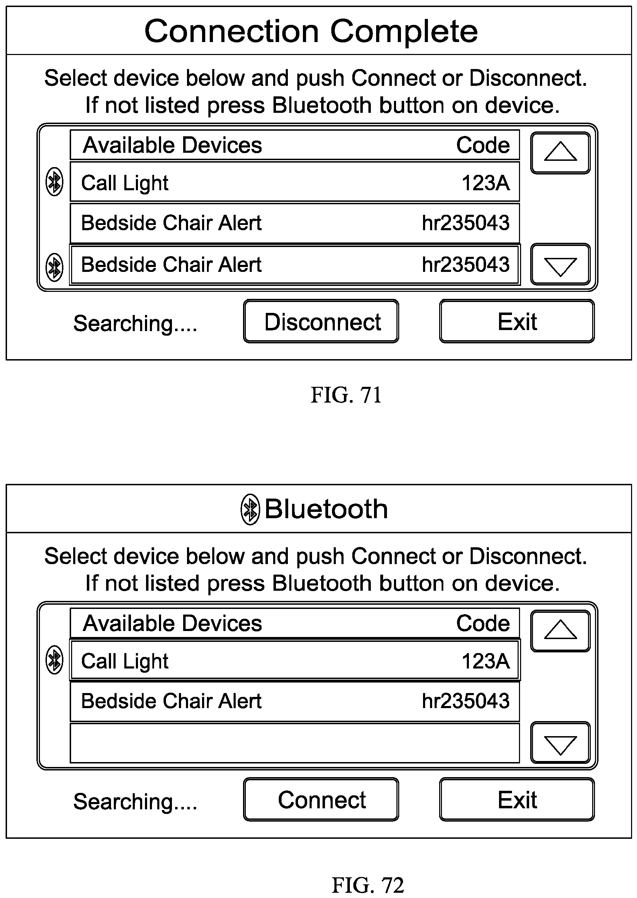

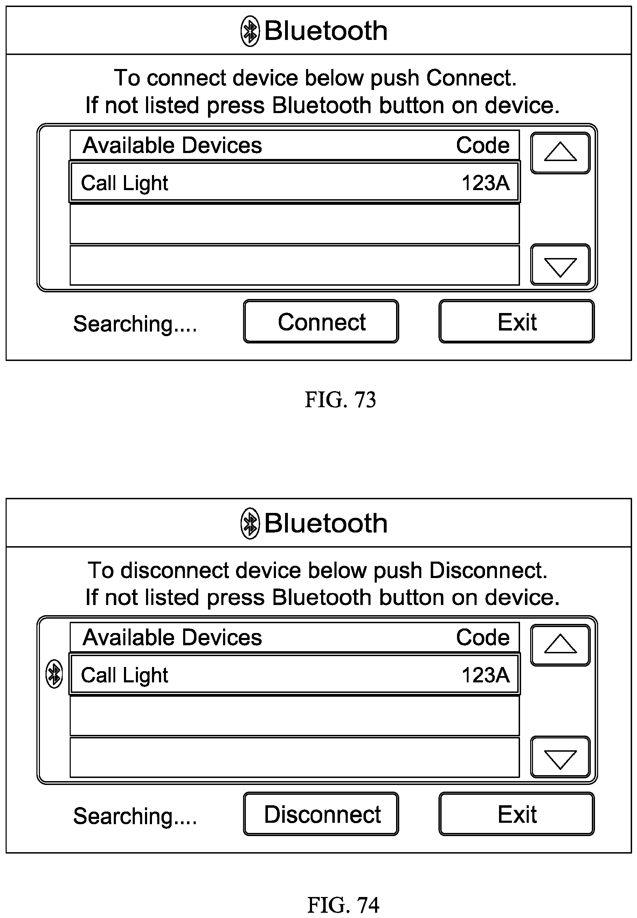

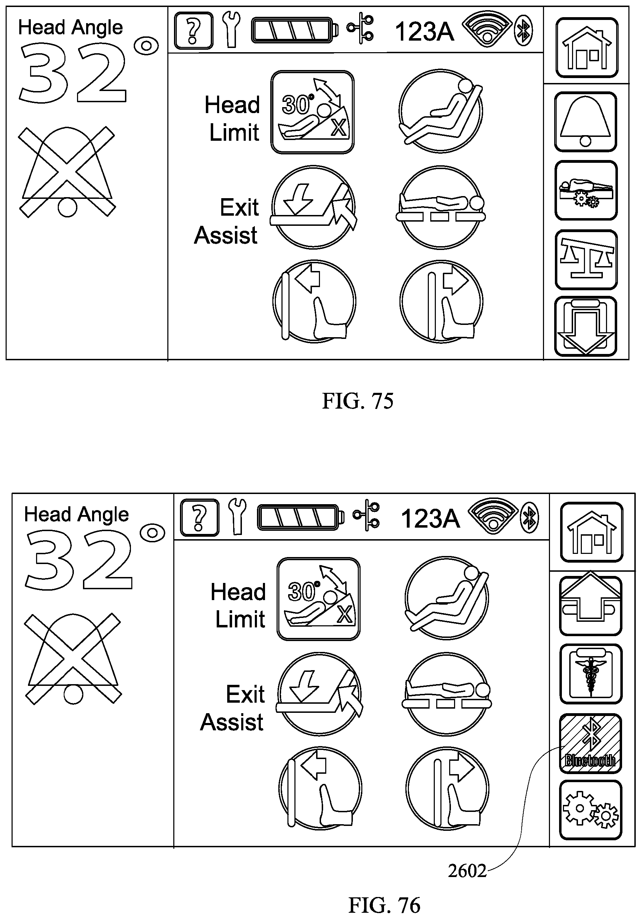

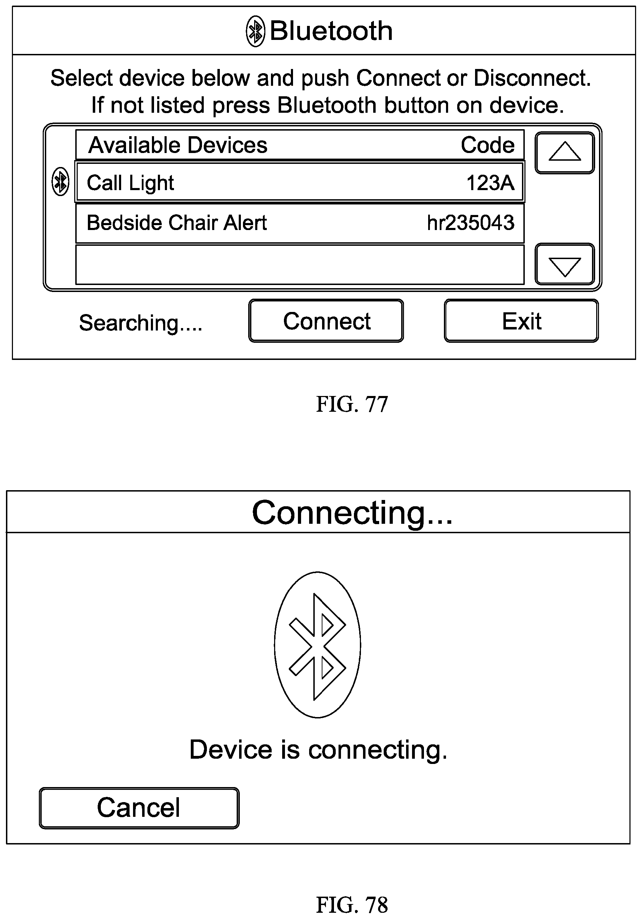

Now referencing the Bluetooth^{®} menu structure 2426, Figs. 71-88 is directed to the Bluetooth^{®} menu structure. The Bluetooth^{®} menu structure starts with Fig. 75, but Fig. 272 does not show the Bluetooth^{®} icon 2602 in the main menu section 2440. Thus a user has to navigate using the navigation arrow in the lower right corner of the screen of Fig. 75 to expose the Bluetooth^{®} icon as shown in Fig. 76. Selection of the Bluetooth^{®} icon 2602 advances to Fig. 77 which provides a listing of available devices showing the call light connected. The user can select one of the devices by a touching the screen in the menu structure will advance to Fig. 78 to connect the device. In some cases, Fig. 71 will appear if another device is searching for Bluetooth^{®} connection. At Fig. 72 the graphical user interface 66 provides prompts to a user for connecting a device.

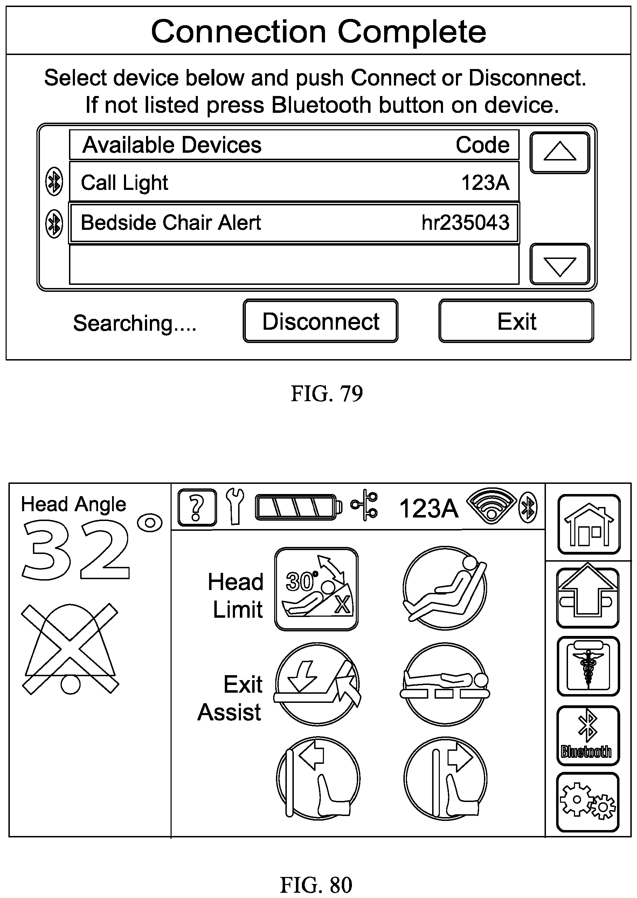

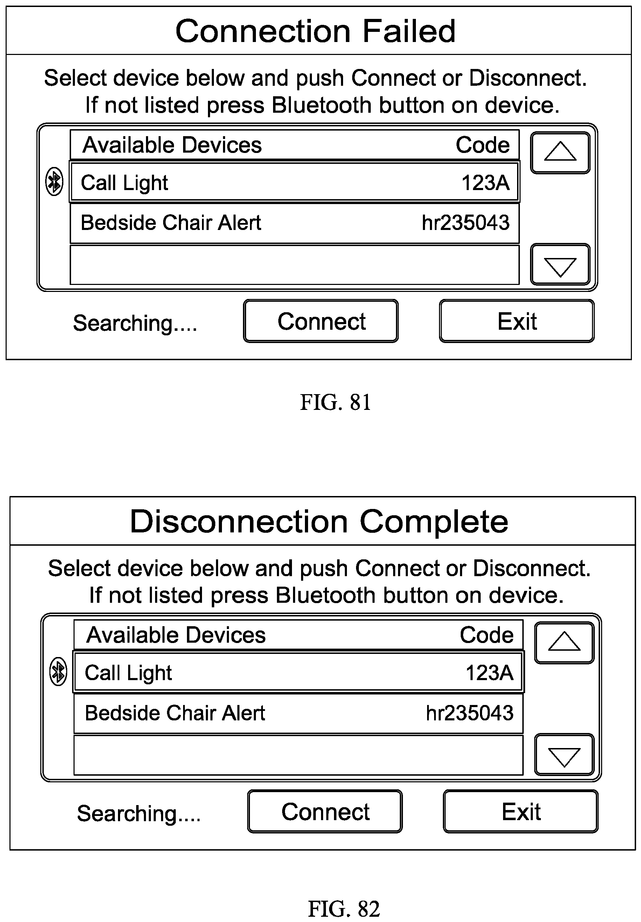

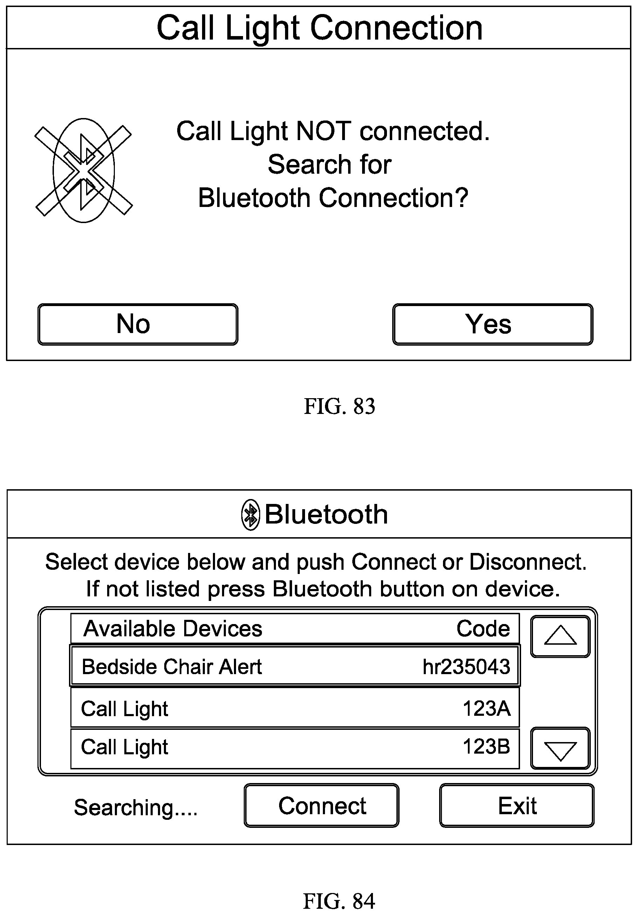

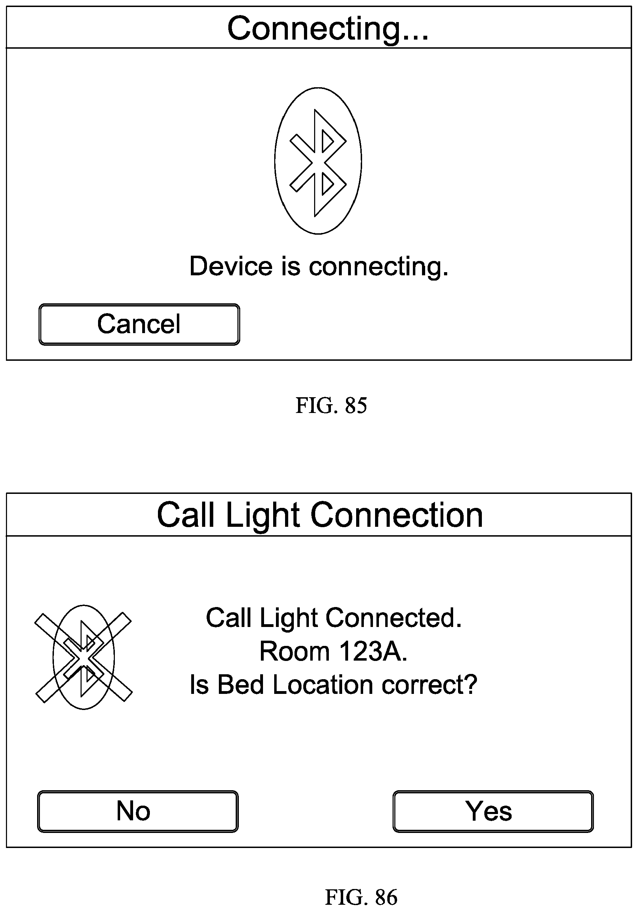

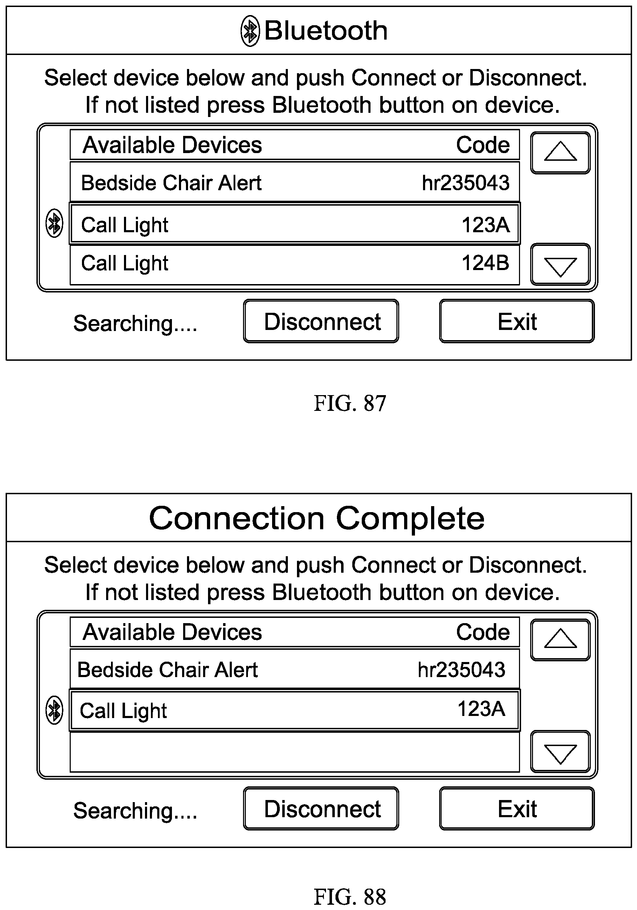

If the connection completes at Fig. 78 the menu structure advances to Fig. 79 which shows other available devices which may be connected or disconnected. Once the Bluetooth^{®} menu structure times out, the home screen shown at Fig. 80 is displayed and displays the Bluetooth^{®} icon if a Bluetooth^{®} connection has been made. If the connection fails at Fig. 78, Fig. 81 provides prompting for resolving the issue. Fig. 82 assists with disconnecting a device. Fig. 83 is a prompt that appears after a bed has been transported to assist with connecting the bed to a Bluetooth^{®} call light. The menu structure then progresses to Fig. 84 to assist with the connection. Fig. 85 indicates the connection is being made and Fig. 88 confirms the completion of the connection. On the other hand, if the bed returns to a room and makes an immediate Bluetooth^{®} connection, a prompt such as that prompt at Fig. 86 appears giving the opportunity to disconnect the device and correctly connected at Fig. 87.

Although certain illustrative embodiments have been described in detail above, variations and modifications exist within the scope as defined in the following claims.

## Claims

1. A patient support apparatus (10) comprising
a controller,
a plurality of sensors, each sensor operable to provide a signal to the controller indicative of the status of a patient position monitoring system of the patient support apparatus (10),
a frame,
a barrier supported by the frame and movable vertically relative to the frame, the barrier comprising a handle;
a control system,
a user interface, and
a notification system (796) coupled to the controller,
wherein the notification system (796) is operable to process signals from the controller which provide an indication of the status of the patient position monitoring system compared to established acceptable operating conditions, and, if the status of the patient position monitoring system deviates from the established acceptable operating condition for the patient position monitoring system, provide a visual indication of the deviation by illuminating the handle in a first manner if the status of the patient position monitoring system is within an acceptable operating condition and in a second manner if the status of the patient position monitoring system is outside acceptable operating condition,
**characterised in that** the handle has a plurality of light emitting diodes positioned within the handle such that emissions from the light emitting diodes are visible external to the handle and **in that** a visual indication of the status of the position of a patient is provided by illuminating the handle.

2. The patient support apparatus (10) of claim 1, wherein a visual indication of the status of a patient position is provided at a foot end (12) of the patient support apparatus (10).

3. The patient support apparatus (10) of claim 1, wherein a visual indication of the status of the patient position is illuminated on a floor under the foot end (12) of the patient support apparatus (10).

4. The patient support apparatus (10) of claim 1, wherein a barrier includes an illuminated handle that provides a nightlight when a patient is determined by the control system of the patient support apparatus (10) to be spaced apart from the patient support apparatus (10).

5. The patient support apparatus (10) of claim 1, wherein a portion of a handle of a barrier illuminates an amber color when a patient position monitoring system is armed and the patient is out of position.

6. The patient support apparatus (10) of claim 1, wherein a visual indication of the status of a condition of at least one feature of the patient support apparatus (10) is provided at a foot end (12) of the patient support apparatus (10).

7. The patient support apparatus (10) of claim 1, wherein a visual indication of the status of a condition of at least one feature of the patient support apparatus (10) is provided by illuminating an indication on the floor under the foot end (12) of the patient support apparatus (10).

8. The patient support apparatus (10) of claim 1, wherein the patient support apparatus (10) is in wireless communication with a sensor operable to detect movement of a patient on a chair adjacent the patient support apparatus (10).

9. The patient support apparatus (10) of claim 8, wherein a chair egress detector communicates wirelessly with a patient position monitoring system of the patient support apparatus (10).

10. The patient support apparatus (10) of claim 9, wherein the chair egress detector arms automatically when a patient sits on the chair egress detector.

11. The patient support apparatus (10) of claim 1, wherein a caregiver interface includes a single button operable to place the patient support apparatus (10) in an optimized egress position.

12. The patient support apparatus (10) of claim 1, wherein placing the patient support apparatus (10) in an optimized egress position includes adjusting deck section members of the patient support apparatus (10).

## Patentansprüche

1. Patientenliegevorrichtung (10), umfassend
eine Steuereinheit,
eine Vielzahl von Sensoren, wobei jeder Sensor dazu dient, ein Signal an die Steuereinheit zu senden, das auf den Status eines Patientenpositionsüberwachungssystems der Patientenliegevorrichtung hinweist (10),
einen Rahmen,
eine Barriere, die von dem Rahmen gestützt wird und senkrecht relativ zum Rahmen bewegt werden kann, wobei die Barriere einen Griff umfasst;
ein Steuersystem,
eine Benutzeroberfläche und
ein an die Steuereinheit gekoppeltes Benachrichtigungssystem (796),
wobei das Benachrichtigungssystem (796) dazu dient, Signale von der Steuereinheit zu verarbeiten, die einen Hinweis auf den Status des Patientenpositionsüberwachungssystems im Vergleich zu feststehenden akzeptablen Betriebsbedingungen bereitstellen, und, falls der Status des Patientenpositionsüberwachungssystems von den feststehenden akzeptablen Betriebsbedingungen für das Patientenpositionsüberwachungssystem abweicht, eine optische Anzeige der Abweichung bereitstellen, indem der Griff auf eine erste Art leuchtet, wenn der Status des Patientenpositionsüberwachungssystems innerhalb einer akzeptablen Betriebsbedingung liegt, und auf eine zweite Art, wenn der Status des Patientenpositionsüberwachungssystems außerhalb einer akzeptablen Betriebsbedingung liegt,
**dadurch gekennzeichnet, dass** der Griff über eine Vielzahl von Leuchtdioden verfügt, die so innerhalb des Griffs positioniert sind, dass Emissionen der Leuchtdioden außerhalb des Griffs sichtbar sind, und dass eine optische Anzeige des Status der Position eines Patienten durch die Beleuchtung des Griffs bereitgestellt wird.

2. Die Patientenliegevorrichtung (10) nach Anspruch 1, wobei eine optische Anzeige des Status einer Patientenposition an einem Fußende (12) der Patientenliegevorrichtung (10) bereitgestellt wird.

3. Die Patientenliegevorrichtung (10) nach Anspruch 1, wobei eine optische Anzeige des Status der Patientenposition auf dem Boden unter dem Fußende (12) der Patientenliegevorrichtung (10) leuchtet.

4. Die Patientenliegevorrichtung (10) nach Anspruch 1, wobei eine Barriere einen beleuchteten Griff beinhaltet, der ein Nachtlicht bereitstellt, wenn das Steuersystem der Patientenliegevorrichtung (10) feststellt, dass sich ein Patient von der Patientenliegevorrichtung (10) entfernt.

5. Die Patientenliegevorrichtung (10) nach Anspruch 1, wobei ein Teil eines Griffs einer Barriere gelb leuchtet, wenn ein Patientenpositionsüberwachungssystem aktiviert ist und sich der Patient an einer anderen Position befindet.

6. Die Patientenliegevorrichtung (10) nach Anspruch 1, wobei eine optische Anzeige des Status einer Bedingung von mindestens einer Funktion der Patientenliegevorrichtung (10) an einem Fußende (12) der Patientenliegevorrichtung (10) bereitgestellt wird.

7. Die Patientenliegevorrichtung (10) nach Anspruch 1, wobei eine optische Anzeige des Status einer Bedingung von mindestens einer Funktion der Patientenliegevorrichtung (10) bereitgestellt wird, indem ein Hinweis auf dem Boden unter dem Fußende (12) der Patientenliegevorrichtung (10) leuchtet.

8. Die Patientenliegevorrichtung (10) nach Anspruch 1, wobei die Patientenliegevorrichtung (10) drahtlos mit einem Sensor kommuniziert, der dazu dient, Bewegung eines Patienten auf einem Stuhl neben der Patientenliegevorrichtung (10) zu erkennen.

9. Die Patientenliegevorrichtung (10) nach Anspruch 8, wobei ein Stuhlausstiegsdetektor drahtlos mit einem Patientenpositionsüberwachungssystems der Patientenliegevorrichtung (10) kommuniziert.

10. Die Patientenliegevorrichtung (10) nach Anspruch 9, wobei der Stuhlausstiegsdetektor automatisch aktiviert wird, wenn ein Patient auf dem Aussteigepositionsdetektor sitzt.

11. Die Patientenliegevorrichtung (10) nach Anspruch 1, wobei eine Benutzeroberfläche für Pflegekräfte eine Einzeltaste beinhaltet, die dazu dient, die Patientenliegevorrichtung (10) in eine optimierte Ausstiegsposition zu positionieren.

12. Die Patientenliegevorrichtung (10) nach Anspruch 1, wobei die Positionierung der Patientenliegevorrichtung (10) in eine optimierte Ausstiegsposition die Anpassung von Teilen der Auflagefläche der Patientenliegevorrichtung (10) beinhaltet.

## Revendications

1. Appareil de support de patient (10) comprenant
un dispositif de commande,
une pluralité de capteurs, chaque capteur fonctionnant pour fournir un signal au dispositif de commande indiquant l'état d'un système de surveillance de position de patient de l'appareil de support de patient (10),
un châssis,
une barrière supportée par le châssis et pouvant être déplacée verticalement par rapport au châssis, la barrière comprenant une poignée ;
un système de commande,
une interface utilisateur et
un système de notification (796) couplé au dispositif de commande,
dans lequel le système de notification (796) peut être utilisé pour traiter les signaux du dispositif de commande qui fournissent une indication de l'état du système de surveillance de position de patient par rapport aux conditions de fonctionnement acceptables établies et, si l'état du système de surveillance de position de patient s'écarte des conditions de fonctionnement acceptables établies pour le système de surveillance de position de patient, fournissent une indication visuelle de l'écart en allumant la poignée d'une première manière si le système de surveillance de position de patient est dans un état de fonctionnement acceptable et d'une seconde manière si le système de surveillance de position de patient est hors des conditions de fonctionnement acceptables,
**caractérisé en ce que** la poignée dispose d'une pluralité de diodes électroluminescentes positionnées à l'intérieur de la poignée de sorte que les émissions des diodes électroluminescentes soient visibles à l'extérieur de la poignée et qu'une indication visuelle de l'état de la position d'un patient soit fournie en éclairant la poignée.

2. Appareil de support de patient (10) selon la revendication 1, dans lequel une indication visuelle de l'état de la position d'un patient est fournie au niveau d'une extrémité de pied (12) de l'appareil de support de patient (10).

3. Appareil de support de patient (10) selon la revendication 1, dans lequel une indication visuelle de l'état de la position du patient est fournie par allumage au sol sous l'extrémité de pied (12) de l'appareil de support de patient (10).

4. Appareil de support de patient (10) selon la revendication 1, dans lequel une barrière comprend une poignée lumineuse fournissant une veilleuse lorsque le système de commande de l'appareil de support de patient (10) détermine qu'un patient doit être éloigné de l'appareil de support de patient (10).

5. Appareil de support de patient (10) selon la revendication 1, dans lequel une partie d'une poignée d'une barrière s'allume en orange lorsqu'un système de surveillance de position de patient est armé et que le patient est hors position.

6. Appareil de support de patient (10) selon la revendication 1, dans lequel une indication visuelle de l'état d'au moins une fonction de l'appareil de support de patient (10) est fournie au niveau d'une extrémité de pied (12) de l'appareil de support de patient (10).

7. Appareil de support de patient (10) selon la revendication 1, dans lequel une indication visuelle de l'état d'au moins une fonction de l'appareil de support de patient (10) est fournie par allumage d'une indication au sol sous l'extrémité de pied (12) de l'appareil de support de patient (10).

8. Appareil de support de patient (10) selon la revendication 1, dans lequel l'appareil de support de patient (10) est en communication sans fil avec un capteur permettant de détecter le mouvement d'un patient sur un siège adjacent à l'appareil de support de patient (10).

9. Appareil de support de patient (10) selon la revendication 8, dans lequel un détecteur de sortie de siège communique sans fil avec un système de surveillance de position de patient de l'appareil de support de patient (10).

10. Appareil de support de patient (10) selon la revendication 9, dans lequel le détecteur de sortie de siège s'arme automatiquement lorsqu'un patient s'assied sur le détecteur de sortie de siège.

11. Appareil de support de patient (10) selon la revendication 1, dans lequel une interface de soignant comprend un bouton unique utilisable pour placer l'appareil de support de patient (10) dans une position de sortie optimale.

12. Appareil de support de patient (10) selon la revendication 1, dans lequel le placement de l'appareil de support de patient (10) dans une position de sortie optimale inclut le réglage des éléments de la section de plateforme de l'appareil de support de patient (10).
